(19) Europäisches Patentamt
European Patent Office
Office européen des brevets

(11) Numéro de publication : 0 427 605 B1

# (12) FASCICULE DE BREVET EUROPEEN

(45) Date de publication du fascicule du brevet : **27.04.94 Bulletin 94/17**

(51) Int. Cl.$^5$ : **C07D 413/12,** A61K 31/535

(21) Numéro de dépôt : **90403123.4**

(22) Date de dépôt : **06.11.90**

## (54) Nouveaux dérivés de la morpholine, leur procédé de préparation et les compositions pharmaceutiques les renfermant.

(30) Priorité : **06.11.89 FR 8914489**

(43) Date de publication de la demande : **15.05.91 Bulletin 91/20**

(45) Mention de la délivrance du brevet : **27.04.94 Bulletin 94/17**

(84) Etats contractants désignés : **AT BE CH DE DK ES FR GB GR IT LI LU NL SE**

(56) Documents cités :
**EP-A- 0 286 495**
**FR-A- 2 073 365**

(73) Titulaire : **ADIR ET COMPAGNIE**
**1 rue Carle Hébert**
**F-92415 Courbevoie Cédex (FR)**

(72) Inventeur : **Regnier, Gilbert**
**Demeure du Plessis, Bâtiment D., 27 av. du Plessis**
**92290 Chatenay Malabry (FR)**
Inventeur : **Guillonneau, Claude**
**21 rue des Bergers**
**F-92140 Clamart (FR)**
Inventeur : **Lepagnol, Jean**
**5 rue de Vlaminck**
**F-78400 Chatou (FR)**
Inventeur : **Lestage, Pierre**
**5 rue des Fontaines du Temple**
**F-75003 Paris (FR)**

(74) Mandataire : **Reverbori, Marcelle**
**Adir et Compagnie, 1, rue Carle Hébert**
**F-92415 Courbevoie Cédex (FR)**

EP 0 427 605 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet les nouveaux dérivés de la morpholine, leur procédé de préparation et les compositions pharmaceutiques les renfermant.

Elle concerne particulièrement les dérivés de la morpholine de formule générale I :

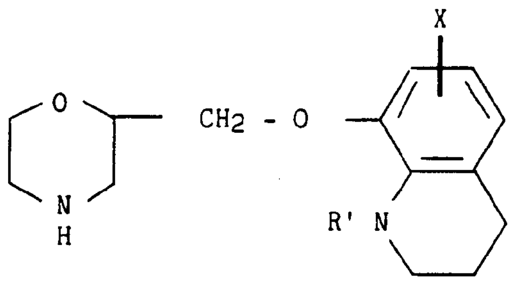

(I)

dans laquelle :

- X, fixé sur le noyau aromatique, représente :
  - un atome d'halogène tel qu'un atome de fluor ou de chlore ou
  - un radical trifluorométhyle ; et
- R' représente :
  - un atome d'hydrogène,
  - un radical alkyle contenant de 1 à 5 atomes de carbone en chaîne droite ou ramifiée, comportant éventuellement une double liaison, ou
  - un radical aralkyle dans lequel la partie alkyle renferme de 1 à 5 atomes de carbone ;
  - sous formes racémique et énantiomères.

L'état antérieur de la technique dans ce domaine est illustré notamment par la demande de brevet européen publiée sous le n° 0286.495 qui concerne des dérivés de la morpholine de formule générale :

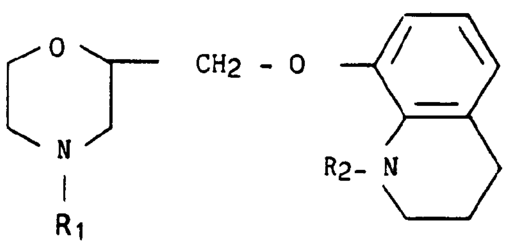

dans laquelle $R_1$ et $R_2$ peuvent représenter, entre autres, un atome d'hydrogène ou un radical alkyle, mais dont la partie aromatique du noyau quinoléine n'est jamais substitué.

L'introduction d'un substituant X sur cette partie aromatique conduit aux dérivés de la présente invention qui se différencient de ceux de la demande européenne 0286.495 non seulement par leur structure chimique mais aussi par l'intensité de leurs effets pharmacologiques et par leur excellente biodisponibilité orale. Ainsi, ils exercent une activité antihypoxique à une dose bien plus faible que celle des dérivés de l'Art antérieur. Cette protection s'exerce par facilitation de la neurotransmission noradrénergique. A la différence des dérivés de l'Art antérieur, les dérivés de la présente invention peuvent ainsi tout autant s'opposer à la défaillance noradrénergique qu'amplifier la libération de noradrénaline induite expérimentalement. Cette facilitation s'exerce à des doses très faibles pour lesquelles les dérivés de l'Art antérieur sont sans effet, et selon une biodisponibilité orale deux à trois fois supérieure à celle des dérivés pris comme produits de référence.

La présente invention a également pour objet le procédé de préparation des dérivés de formule générale I caractérisé en ce que :

**A)** Dans le cas où R' représente un atome d'hydrogène :

- l'on fait réagir une morpholine substituée de formule générale II :

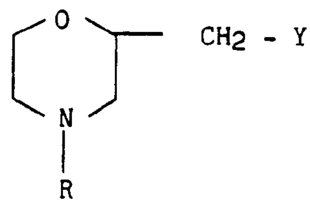

(II)

dans laquelle :

- R est un groupe protecteur tel qu'un groupe benzyle ou trityle et
- Y est un atome de chlore ou de brome ou un reste tosyloxy,

avec un sel alcalin de l'hydroxyquinoléine de formule générale III :

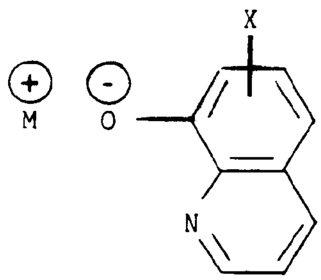

(III)

dans laquelle :

- X a la signification définie précédemment et
- M est un métal alcalin tel que sodium ou potassium ;
- et l'on réduit le dérivé ainsi obtenu de formule générale IV :

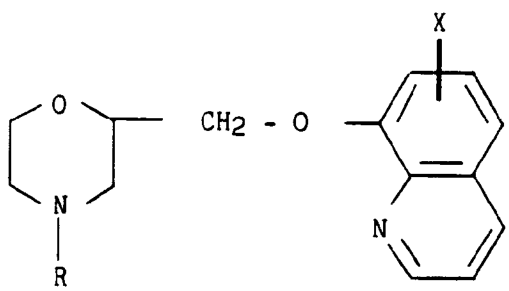

(IV)

dans laquelle R et X ont les significations précédemment définies ;

afin d'obtenir les dérivés I dans le cas où R' est un atome d'hydrogène, soit les dérivés répondant plus précisément à la formule générale I' :

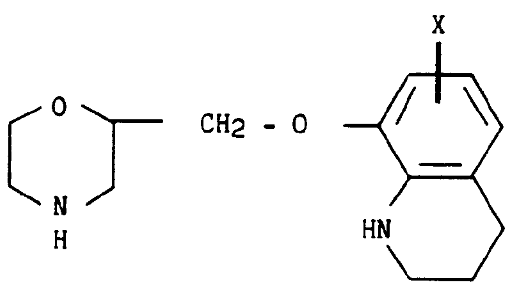

(I')

dans laquelle X a la signification précédemment définie ; et

**B)** dans le cas où R' représente :

- un radical alkyle contenant de 1 à 5 atomes de carbone en chaîne droite ou ramifiée, comportant éventuellement une double liaison, ou
- un radical aralkyle dans lequel la partie alkyle renferme de 1 à 5 atomes de carbone :
  - l'on réduit le composé de formule IV précédemment définie, la réduction s'effectuant au moyen d'un hydroborure, (tel que $NaBH_3CN$) comme décrit ci-dessus ;
  - puis l'on alkyle le composé intermédiaire de formule générale V :

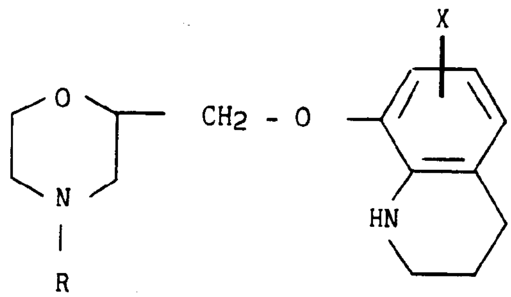

(V)

dans laquelle X et R ont la signification précédemment définie ;
l'alkylation étant effectuée :

. soit au moyen d'un aldéhyde de formule R"-CHO dans laquelle R" représente :
  - un atome d'hydrogène,
  - un radical alkyle ayant de 1 à 4 atomes de carbone en chaîne droite ou ramifiée comportant éventuellement une double liaison, ou
  - un radical aralkyle dans lequel la partie alkyle renferme de 1 à 4 atomes de carbone, et de $Na BH_3 CN$,

. soit au moyen d'un acide de formule R"COOH dans laquelle R" a la signification définie ci-dessus, et
de $Na BH_4$,

- et enfin l'on débenzyle ou l'on détrityle les composés obtenus de formule générale VI :

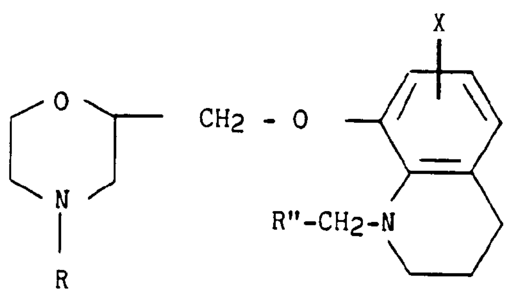

(VI)

dans laquelle X, R et R" ont les significations précédemment définies, en opérant comme décrit précédemment (pour préparer les dérivés I'),

afin d'obtenir les dérivés I dans le cas où R' représente :

- un radical alkyle contenant de 1 à 5 atomes de carbone en chaîne droite ou ramifiée comportant éventuellement une double liaison, ou
- un radical aralkyle dans lequel la partie alkyle renferme de 1 à 5 atomes de carbone,

soit, les dérivés répondant plus précisément à la formule générale I" :

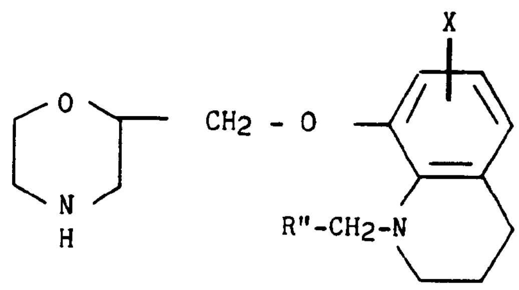

(I")

dans laquelle X et R" ont les significations précédemment définies.

La condensation des dérivés II et III est réalisée de façon particulièrement adéquate en opérant dans un solvant aprotique polaire, tel que par exemple la diméthyl formamide ou la diméthyl acétamide.

On peut également effectuer la condensation du dérivé II avec le dérivé III à l'état de sel de sodium ou de potassium, par transfert de phase solide-liquide dans un solvant aprotique tel qu'un hydrocarbure aromatique comme par exemple le toluène ou le xylène, en présence d'un sel d'ammonium quaternaire à haut poids moléculaire, tel que par exemple l'adogène, à des températures comprises entre 90 et 120°C.

La réduction du dérivé IV s'effectue dans le cas où R est un radical benzyle au moyen d'hydrogène, à basse pression (de 2 à 5 atmosphères) en présence d'un catalyseur appartenant aux métaux du VIIIème groupe tels que le rhodium ou le palladium sur charbon, (le palladium pouvant également être utilisé sous forme d'hydroxyde), dans un solvant polaire tels que les alcools à bas poids moléculaire, à une température comprise entre 20 et 60°C. Dans ce cas, il y a élimination concomitante du groupe benzyle protecteur.

Si l'on veut éviter l'emploi d'hydrogène, on peut réaliser la réduction du dérivé IV au moyen d'un hydroborure. La méthode la plus adéquate consiste, à partir d'un dérivé de formule IV dans laquelle R représente un radical trityle, lequel par hydrolyse ménagée au moyen d'un acide minéral ou organique permet d'obtenir le dérivé de formule IV dans laquelle R est remplacé par un atome d'hydrogène ; ce dernier étant réduit d'une manière classique par un hydroborure, tel que $NaBH_3CN$, dans un solvant polaire comme le méthanol ou l'éthanol pur ou en mélange avec de l'eau, à un pH compris entre 6 et 7.

L'alkylation des dérivés V au moyen de R"-CHO et de $NaBH_3CN$ est effectuée dans un solvant aprotique polaire pur (comme par exemple $CH_3CN$) ou en mélange avec de l'eau, à pH contrôlé selon la technique décrite par R.BORCH et coll. J. am. chem. soc. 93, 2897 (1971).

L'alkylation des dérivés V au moyen de R"COOH et de $NaBH_4$ est effectuée dans un solvant aprotique polaire (comme par exemple le tétrahydrofuranne ou le dioxane) selon la technique décrite par GRIBBLE et HEALD, Synthesis (1975), 650.

Les dérivés de formule générale I donnent des sels avec les acides physiologiquement tolérables - sels qui sont, à ce titre, inclus dans la présente invention.

Les dérivés de la présente invention possèdent des propriétés pharmacologiques et thérapeutiques intéressantes. Ils s'opposent en particulier aux désordres cérébraux créés par une baisse d'apport en oxygène. Leur mécanisme d'action principal est la facilitation de la neurotransmission noradrénergique, neurotransmission étroitement impliquée dans les phénomènes d'attention, de vigilance et de mémorisation, et dont le dysfonctionnement est largement démontré lors de pathologies cérébrales telles que le vieillissement, la dépression ou l'accident vasculaire.

Ces propriétés intéressantes s'excercent de manière plus intense et avec beaucoup plus de sécurité pharmacotoxicologique avec les dérivés de la présente invention qu'avec les dérivés de la demande de brevet européen n° 0286.495 dont le dérivé de l'exemple 2, dénommé R,S [(morpholinyl-2) méthoxy]-8 tétrahydro-1,2,3,4 quinoléine, était le représentant le plus actif, comme le prouve l'étude pharmacologique ci-après décrite dans l'exemple 8.

Ainsi, les composés de la présente invention s'avèrent utiles pour le traitement thérapeutique des syndromes liés à une baisse d'oxygénation et à une défaillance de la neurotransmission noradrénergique, tels que l'accident vasculaire cérébral ou le vieillissement cérébral.

Ces effets bénéfiques leur permettent de protéger les neurones et d'améliorer les phénomènes de baisse d'attention, de concentration et de vigilance ainsi que les états dépressifs et amnésiques qui accompagnent toujours l'ischémie et les maladies dégénératives cérébrales ainsi que le vieillissement.

La présente invention a également pour objet les compositions pharmaceutiques contenant comme principe actif un composé de formule générale I ou un de ses sels physiologiquement tolérable, mélangé ou associé à un excipient pharmaceutique approprié, comme par exemple le glucose, le lactose, l'amidon, le talc, l'éthylcellulose, le stéarate de magnésium ou le beurre de cacao.

Les compositions pharmaceutiques ainsi obtenues se présentent généralement sous forme dosée et peuvent contenir de 0,5 à 100 mg de principe actif. Elles peuvent revêtir, par exemple, la forme de comprimés, dragées, gélules, suppositoires, solutions injectables ou buvables et être, selon les cas, administrées par voie orale, rectale ou parentérale à la dose de 0,5 à 100 mg en 1 à 3 prises journalières.

Les exemples suivants illustrent la présente invention, les points de fusion étant déterminés à la platine chauffante de Kofler, sauf mention contraire.

**EXEMPLE 1 :**

R,S fluoro-6 (morpholinyl-2 méthoxy)-8 tétrahydro-1,2,3,4 quinoléine.

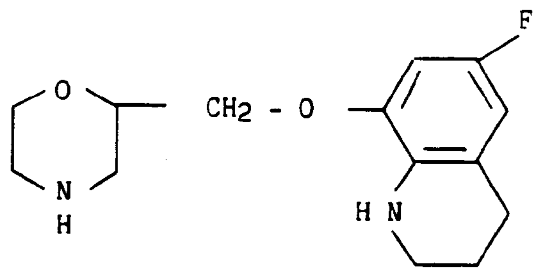

**a)** Préparation de :

R,S fluoro-6 (benzyl-4 morpholinyl-2 méthoxy)-8 quinoléine :

Dans un ballon muni d'une agitation très efficace et d'un réfrigérant, on introduit 9,54 g de sel de sodium de la fluoro-6 hydroxy-8 quinoléine, fondant à 134°C, 19 g de R,S tosyloxyméthyl-2 benzyl-4 morpholine, fondant à 74°C, 1,2 g d'adogène 464 et 250 ml de toluène. On porte à reflux 7 heures. On ajoute alors 10 g supplémentaires de R,S tosyloxyméthyl-2 benzyl-4 morpholine et maintient le reflux pendant encore 16 heures.

Après refroidissement, on filtre et lave le filtrat 4 fois avec de la soude normale. On sèche sur sulfate de sodium et concentre à sec. Le résidu est chromatographié sur 1,6 l de silice (35-70 μ). On élue à l'acétate d'éthyle. Les fractions retenues sont concentrées à sec. On obtient 11,9 g de résidu gommeux. Rendement : 65,5 %.

Le sel de sodium de la fluoro-6 hydroxy-8 quinoléine de départ a été préparé comme suit :

On dissout 2,06 g de soude en pastilles dans 2,5 ml d'eau. On ajoute 150 ml de méthanol et 8,39 g de fluoro-6 hydroxy-8 quinoléine. On agite jusqu'à dissolution, puis concentre à sec et sèche à 50°C sous 0,5 torr. On obtient 9,54 g de sel de sodium de la fluoro-6 hydroxy-8 quinoléine, avec un rendement de 100 %.

**b)** Réduction de :

R,S fluoro-6 (benzyl-4 morpholinyl-2 méthoxy)-8 quinoléine :

Dans un flacon en acier inox, on introduit 11,2 g de R,S fluoro-6 (benzyl-4 morpholinyl-2 méthoxy)-8 quinoléine, 400 ml de méthanol, 62 ml d'acide chlorhydrique N et 1,12 g de palladium sur charbon à 10 %. On hydrogène à 60°C sous une pression de 6 kg pendant 3 heures. On filtre le palladium et le remplace par la même quantité du même catalyseur. On hydrogène à nouveau à 60°C sous une pression de 6 kg pendant 3 heures. On remplace une dernière fois le catalyseur par une même quantité du même catalyseur frais et hydrogène à nouveau à 60°C, sous 6 kg pendant 3 heures. On filtre et concentre à sec le filtrat. Le résidu est repris dans une solution à 10 % de carbonate de sodium. On extrait plusieurs fois au chlorure de méthylène et l'on sèche sur sulfate de sodium.

On concentre à sec et obtient 7,4 g de résidu que l'on dissout dans 74 ml d'éthanol. On ajoute 2,67 g d'acide méthanesulfonique. On observe une cristallisation. On essore, lave les cristaux à l'éthanol glacé et sèche à 70°C sous 0,5 torr. On obtient 8,5 g de monométhanesulfonate de R,S fluoro-6 (morpholinyl-2 méthoxy)-8 tétrahydro-1,2,3,4 quinoléine, fondant à 189°C. Rendement : 80 %.

En opérant comme ci-dessus décrit dans l'exemple 1, à partir de la fluoro-6 hydroxy-8 quinoléine et respectivement :

la R tosyloxy-méthyl-2 benzyl-4 morpholine,

P.F. : 72°C, $\alpha_D^{21}$ = -19,9° (c = 2, $CH_3$ OH),

et la S tosyloxyméthyl-2 benzyl-4 morpholine,

P.F. : 72°C, $\alpha_D^{21}$ = +21° (c = 2, $CH_3$ OH),

on obtient la R et la S fluoro-6 (benzyl-4 morpholinyl-2 méthoxy)-8 quinoléine, lesquelles, par hydrogénation donnent respectivement : la R et la S fluoro-6 (morpholinyl-2 méthoxy)-8 tétrahydro-1,2,3,4 quinoléine, avec lesquelles on obtient respectivement :

α) le monométhane sulfonate de R fluoro-6 (morpholinyl-2 méthoxy)- 8 tétrahydro-1,2,3,4 quinoléine, P.F.(cap):190-191°C, $\alpha_D^{21}$ = -4,8° (c=1, DMSO), et

β) le monométhane sulfonate de S fluoro-6 (morpholinyl-2 méthoxy)-8 tétrahydro-1,2,3,4 quinoléine, P.F.(cap): 191-192°C, $\alpha_D^{21}$ =+3,3°(c=1,DMSO).

**Exemple 2 :**

R,S fluoro-5 (morpholinyl-2 méthoxy)-8 tétrahydro-1,2,3,4 quinoléine :

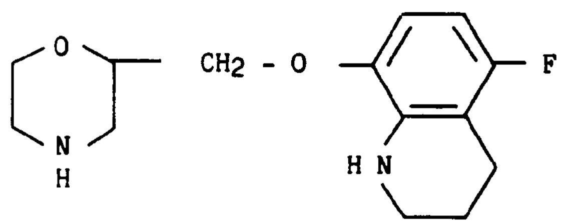

**a)** Préparation de :

R,S fluoro-5 (trityl-4 morpholinyl-2 méthoxy)-8 quinoléine :

Dans un ballon tricol muni d'une agitation et d'un réfrigérant très efficace, on introduit 8,87 g de sel de sodium de la fluoro-5 hydroxy-8 quinoléine, fondant à 114°C, 27,1 g de R,S tosyloxyméthyl-2 trityl-4 morpholine, fondant à 252°C, 1 g d'adogène 464 et 250 ml de toluène. On porte au reflux pendant 20 heures sous agitation, puis filtre et concentre à sec.

On chromatographie sur 2,1 l de silice (AMICON - (35-70 μ) en éluant avec un mélange de chlorure de méthylène et d'acétate d'éthyle (95 - 5). Les fractions retenues sont concentrées à sec, reprises dans le chlorure de méthylène et lavées trois fois à la soude N afin d'éliminer les traces de matières premières.

On sèche sur sulfate de sodium et concentre à sec. On obtient 16,6 g de R,S fluoro-5 (trityl-4 morpholinyl-2 méthoxy)-8 quinoléine, sous forme d'une gomme épaisse. Rendement : 69 %.

Le sel de sodium de la fluoro-5 hydroxy-8 quinoléine a été préparé par la même méthode que son homologue fluoro-6, (cf. fin de l'exemple 1a).

**b)** Préparation de :

R,S fluoro-5 (morpholinyl-2 méthoxy)-8 quinoléine :

13,74 g de R,S fluoro-5 (trityl-4 morpholinyl-2 méthoxy)-8 quinoléine précédemment préparée, sont agités dans 200 ml d'acide chlorhydrique normal et 250 ml d'éther pendant 3 heures de façon très efficace. La phase aqueuse est décantée et lavée une fois à l'éther. On alcalinise par du carbonate de sodium en poudre. On extrait au chlorure de méthylène, sèche la phase organique sur sulfate de sodium et concentre à sec. On obtient 6,6 g de R,S fluoro-5 (morpholinyl-2 méthoxy)-8 quinoléine sous forme d'une gomme. Rendement : 92,5 %.

**c)** Réduction de :

R,S fluoro-5 (morpholinyl-2 méthoxy)-8 quinoléine :

Dans un ballon tricol muni d'une agitation, d'un thermomètre, d'un réfrigérant et d'une ampoule de coulée, on introduit 6,6 g de R,S fluoro-5 (morpholinyl-2 méthoxy)-8 quinoléine et 250 ml de méthanol.

On ajoute avec précaution 6,3 g de cyanoborohydrure de sodium. La température s'élève de 20 à 35°C. On porte au reflux et ajoute, en une heure, 100 ml d'acide chlorhydrique normal.

On effectue ensuite une seconde introduction alternée de cyanoborohydrure de sodium et d'acide chlorhydrique normal dans les mêmes conditions de temps, de température et de quantité que précédemment.

On maintient le reflux pendant 10 minutes après la fin de la seconde introduction ; puis on concentre à sec. On reprend le résidu dans un mélange de chlorure de méthylène et de solution aqueuse de carbonate de sodium à 10 %.

On extrait plusieurs fois la phase aqueuse au chlorure de méthylène. Les phases organiques jointes sont séchées sur sulfate de sodium et concentrées à sec. Le résidu est chromatographié sur 500 ml de silice AMICON (35-70 μ) sous 1 bar de pression, en éluant avec un mélange de chlorure de méthylène et de méthanol (80 - 20). Les fractions retenues sont concentrées à sec.

On obtient 4,8 g de R,S fluoro-5 (morpholinyl-2 méthoxy)-8 tétrahydro-1,2,3,4 quinoléine sous forme de gomme épaisse. On reprend cette gomme dans 18 ml d'éthanol chaud. On ajoute un mélange contenant 1,73 g d'acide méthanesulfonique dissous dans 30 ml d'éthanol.

On observe une cristallisation. On essore les cristaux, lave à l'éthanol glacé et sèche à 70°C, sous 10 torr.

On obtient 5,7 g de monométhane sulfonate de R,S fluoro-5 (morpholinyl-2 méthoxy)-8 tétrahydro-1,2,3,4 quinoléine, P.F. : 201°C. Rendement : 63 %.

En opérant comme ci-dessus décrit dans l'exemple 2 a), à partir de la fluoro-5 hydroxy-8 quinoléine et respectivement :

la R tosyloxyméthyl-2 trityl-4 morpholine,
P.F. 250°C, $\alpha_D^{21}$ = +17° (c = 1, $CH\ Cl_3$),

et la S tosyloxyméthyl-2 trityl-4 morpholine,
P.F. : 250°C, $\alpha_D^{21}$ = -15,8° (c = 1, $CH\ Cl_3$),

on obtient respectivement :

la R fluoro-5 (trityl-4 morpholinyl-2 méthoxy)-8 quinoléine,
$\alpha_D^{20°5}$ = +67,9° (c = 1, $CH\ Cl_3$),

et la S fluoro-5 (trityl-4 morpholinyl-2 méthoxy)-8 quinoléine,
$\alpha_D^{21}$ = -68° (c=1,$CH\ Cl_3$),

qui traitées selon la méthode décrite dans l'exemple 2 b) donnent respectivement :

la R fluoro-5 (morpholinyl-2 méthoxy)-8 quinoléine,
$\alpha_D^{22}$ = +4,4° (c = 1, $C_2H_5\ OH$),

et la S fluoro-5 (morpholinyl-2 méthoxy)-8 quinoléine,
$\alpha_D^{21}$ = -4,7° (c = 1, $C_2H_5\ OH$),

lesquelles par réduction donnent respectivement :

la R et la S fluoro-5 (morpholinyl-2 méthoxy)-8 tétrahydro-1,2,3,4 quinoléine. avec lesquelles on obtient respectivement :

α) le monométhane sulfonate de R fluoro-5 (morpholinyl-2 méthoxy)-8 tétrahydro-1,2,3,4 quinoléine, P.F.(cap):193-194°C, $\alpha_D^{21}$ = + 4,55° (c= 1, $C_2H_5OH$), et

β) le monométhane sulfonate de S fluoro-5 (morpholinyl-2 méthoxy)-8 tétrahydro-1,2,3,4 quinoléine, P.F.(cap): 193-194°C, $\alpha_D^{21}$ = -4,75°(c=1,$C_2H_5OH$).

**Exemples 3 et 4:**

En opérant selon la méthode décrite, dans l'exemple 2 ont été préparées :

3) la R,S chloro-5 (morpholinyl-2 méthoxy)-8 tétrahydro-1,2,3,4 quinoléine, (P.F. du monométhanesulfonate correspondant :180°C), et les énantiomères R et S correspondants, et

4) la R,S trifluorométhyl-5(morpholinyl-2 méthoxy)-8 tétrahydro-1,2,3,4 quinoléine, P.F. (cap) : 101-103°C, P.F. (cap) du monométhane sulfonate correspondant : 178-180°C,[à partir de la trifluorométhyl-5 hydroxy-8 quinoléine, P.F. : 98°C] et les énantiomères R et S correspondants.

**Exemple 5 :**

R,S fluoro-6 méthyl-1 (morpholinyl-2 méthoxy)-8 tétrahydro-1,2,3,4 quinoléine.

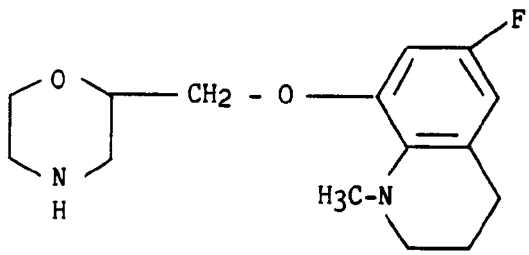

**a)** A une solution de 4,7 g de fluoro-6(benzyl-4 morpholinyl-2 méthoxy)-8 quinoléine (préparée selon l'exemple 1a) dans 30 ml d'acétonitrile, on ajoute 5,3 ml de formol à 40 % et 1,3 g de cyanoborohydrure de sodium puis 0,66 ml d'acide acétique (pH=5).

On observe une élévation de température ; on refroidit à 20°C et agite deux heures à cette température.

On évapore à sec, reprend le résidu par $CH_2Cl_2$ et NaOH N et extrait plusieurs fois au $CH_2Cl_2$. Après évaporation du solvant on chromatographie sur 2,5 kg de silice (35-70μ) en éluant avec le mélange $CH_2Cl_2$-acétone (9-1). On recueille, après évaporation des éluats, 3,7 g de produit sous forme d'huile épaisse.

**b)** 3,6 g de R,S fluoro-6 méthyl-1(benzyl-4 morpholinyl-2 méthoxy)-8 tétrahydro-1,2,3,4 quinoléine, précédemment obtenus, sont dissous dans 70 ml d'éthanol. On ajoute 20 ml d'HCl N et on hydrogène sous 6 atmosphères d'hydrogène en présence de 0,5 g de $Pd(OH)_2$ (catalyseur de Pearlman) à 60°C. Au bout de 20 heures, l'hydrogénolyse est terminée. On filtre le catalyseur et évapore le solvant. Le résidu est extrait au $CH_2Cl_2$. On lave plusieurs fois à l'eau, décante et évapore. On obtient 2,3 g de produit résineux qui est transformé en méthanesulfonate dans l'éthanol. On obtient ainsi 2,3 g de méthanesulfonate de R,S fluoro-6 méthyl-1 (morpholinyl-2 méthoxy)-8 tétrahydro-1,2,3,4 quinoléine.
En opérant de la même façon, ont été préparés les énantiomères R et S correspondants.

**Exemples 6 et 7 :**

En opérant selon la méthode décrite dans l'exemple 5 ont été préparées :
6) la R,S fluoro-5 méthyl-1 (morpholinyl-2 méthoxy)-8 tétrahydro-1,2,3,4 quinoléine, [à partir de la fluoro-5 (trityl-4 morpholinyl-5 méthoxy)-8 quinoléine, préparée selon l'exemple 2a] et les énantiomères R et S correspondants, et
7) la R,S trifluorométhyl-5 méthyl-1 (morpholinyl-2 méthoxy)-8 tétrahydro-1,2,3,4 quinoléine, son monométhane sulfonate P.F.(cap): 122-124°C, [à partir de la trifluorométhyl-5 (benzyl-4 morpholinyl-2 méthoxy)-8 quinoléine, P.F.(cap) : 130-132°C] et les énantiomères R et S correspondants.

**Exemple 8 :**

Etude pharmacologique :

Les dérivés de la présente invention ont été testés comparativement au produit le plus actif de l'état antérieur de la technique à savoir la R,S [(morpholinyl-2) méthoxy]-8 tétrahydro-1,2,3,4 quinoléine (objet de l'exemple 2 de la demande de brevet européen n° 0286.495) ci-après dénommé : produit de référence.

**1. Effets protecteurs cérébraux chez la souris :**

Des souris mâles sont soumises à une hypoxie globale aigüe, dans un caisson à dépression barométrique, où la pression est abaissée rapidement à 160 mbar. Le temps de survie cérébrale objectivée par l'apparition du dernier gasp respiratoire est mesuré chez des animaux témoins et chez des animaux recevant 30 minutes auparavant, par voie intrapéritonéale, le composé étudié.

Dans ces conditions, les dérivés de l'invention s'opposent significativement à l'hypoxie et retardent la mort cérébrale dès la dose de 3 μmoles/kg.

Par exemple, à cette dose, on observe une augmentation du temps de survie de 18% ($p < 0,03$) avec le produit de l'exemple 1α et de 21 % ($p < 0,05$) avec le produit de l'exemple 2β. Le même effet protecteur avec le produit de référence n'est obtenu qu'à la dose de 9 μmoles/kg.

Lorsque les composés sont administrés par voie orale, 60 minutes avant l'hypoxie, le produit de l'exemple 1α augmente le temps de survie de 37% à la dose de 9 μmoles/kg tandis que le produit de référence l'augmente

de 31% à la dose de 40 µmoles/kg. Les dérivés de la présente invention ont donc une excellente biodisponibilité orale même aux très faibles doses étudiées, puisque le rapport entre les doses actives par voie PO et IP est compris entre 2 et 3.

L'effet antihypoxique des dérivés de l'invention est également lié à la dose, comme le montrent, par exemple, les résultats suivants :

| Produits testés | DOSES (µ moles/kg I.P.) | | | |
|---|---|---|---|---|
| | 3 | 9 | 30 | 90 |
| exemple 1α<br>exemple 2β | + 18%<br>+ 21% | + 37%<br>+ 42% | + 55%<br>+ 62% | + 114%<br>+ 108 |
| Produit de référence | + 5 % | + 17 % | + 42 % | + 87 % |

## 2. Effets facilitateurs noradrénergiques in vivo

### a) Test de la létalité à la yohimbine chez la souris

Des souris mâles reçoivent par voie intrapéritonéale, de la yohimbine (antagoniste $\alpha_2$) à la dose de 30mg/kg, dose non létale qui n'entraîne que l'apparition de symptômes d'hyperexcitabilité ou de convulsions. Tout composé facilitant la neurotransmission noradrénergique, potentialise l'effet libérateur de la yohimbine et entraine la mort des animaux.

Un pourcentage de mortalité est ainsi mesuré pour chaque groupe d'animaux traités 30 minutes auparavant par voie IP ou 60 minutes auparavant par voie PO avec le composé étudié.

Dans ces conditions, les dérivés de la présente invention exercent un effet facilitateur noradrénergique marqué dès la dose de 9 µmoles/kg IP (mortalité=15 à 30%). Cet effet est identique à celui du produit de référence (+ 20%) mais la biodisponibilité orale, exprimée par le rapport entre les doses efficaces moyennes PO et IP, s'avère bien meilleure pour les dérivés de l'invention. Ce rapport est en effet de 4,25 pour le produit de référence, alors qu'il n'est que de 3,16 pour le produit de l'exemple 1α et de 1,77 pour le produit de l'exemple 2α.

### b) Hypothermie à l'apomorphine chez la souris

L'administration d'apomorphine à très forte dose (16mg/kg IP) entraîne une hypothermie d'environ 4°C par stimulation d'hétérorécepteurs dopaminergiques situés sur les neurones noradrénergiques. L'hypothermie ainsi créée est donc due à la défaillance de la neurotransmission noradrénergique. Les agents facilitateurs administrés 30 minutes auparavant s'opposent à cette hypothermie.

Dans ces conditions, les dérivés de l'invention exercent un effet inhibiteur vis-à-vis de l'hypothermie dès la dose de 3 µmoles/kg (Produit de l'exemple 2β:-16% ; Produit de l'exemple 1:-27%) alors que le produit de référence reste sans effet. Il en est de même à la dose de 9 µmoles/kg (Produit de l'exemple 2β:-21% ; Produit de l'exemple 1:-46% ; Produit de l'exemple 2:-29% , Produit de référence: + 0,5%)

## 3. Effet facilitateur noradrénergique in vitro

Cet effet a été recherché dans l'hippocampe par mesure de la recapture synaptosomale de la noradrénaline (NAD). Pour cela, une fraction de synaptosomes est mise en présence de NAD tritiée avec ou sans incubation préalable avec le composé étudié.

La mesure de la radioactivité dans le culot synaptosomal permet d'apprécier la quantité de NAD recaptée naturellement par les terminaisons neuronales. Un composé inhibant la recapture diminue la radioactivité mesurée et un pourcentage d'inhibition est mesuré par rapport à une préparation témoin.

A la dose de $10^{-6}$M, le produit de référence inhibe de 33,6% la recapture de NAD. Les dérivés de l'invention s'opposent plus intensément à cette recapture (Produit de l'exemple 1β:-55,4% ; Produit de l'exemple 2β :-

58,9%).

**4. Effets psychoéveillants chez la souris**

Des souris mâles reçoivent par voie IP, du barbital sodique à la dose de 270 mg/kg. La durée de la narcose ainsi induite est comparée entre des animaux témoins et des animaux traités par voie IP au préalable par les composés étudiés.

A la dose de 30 µmoles/kg, le produit de référence s'oppose significativement à la narcose au barbital (-20%, p <0,05). Dans les mêmes conditions, et à la même dose, les dérivés de l'invention exercent un effet marqué (Produit de l'exemple 2β :-40%, p <0,02 ; Produit de l'exemple 1 :-36%, p<0,01).

Cet antagonisme de la narcose au barbital reflète un effet psychoéveillant puisqu'il n'apparaît pas d'antagonisme lorsque la narcose est induite à l'hexobarbital (75mg/kg IP) (Produit de l'exemple 2β:+20% ; Produit de l'exemple 1:+26%).

## Revendications

**Revendications pour les Etats contractants suivants : AT, BE, CH, DE, DK, FR, GB, IT, LI, LU, NL, SE**

1. Les dérivés de la morpholine de formule générale (I) :

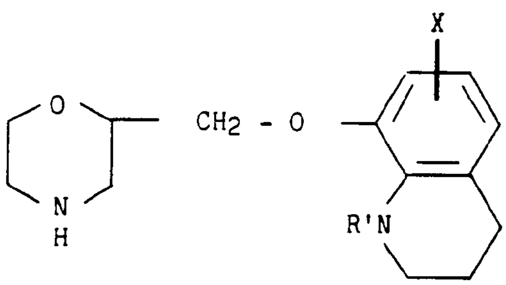

(I)

dans laquelle :
- X, fixé sur le noyau aromatique, représente :
  - un atome d'halogène tel qu'un atome de fluor ou de chlore, ou
  - un radical trifluorométhyle ; et
- R' représente :
  - un atome d'hydrogène,
  - un radical alkyle contenant de 1 à 5 atomes de carbone en chaîne droite ou ramifiée comportant éventuellement une double liaison, ou
  - un radical aralkyle dans lequel la partie alkyle renferme de 1 à 5 atomes de carbone ;
  sous formes racémique et énantiomères.

2. Les sels physiologiquement tolérables des composés de la revendication 1 avec des acides appropriés.

3. La R,S fluoro-6 (morpholinyl-2 méthoxy)-8 tétrahydro-1,2,3,4 quinoléine et ses énantiomères.

4. La R,S fluoro-5 (morpholinyl-2 méthoxy)-8 tétrahydro-1,2,3,4 quinoléine et ses énantiomères.

5. La R,S chloro-5 (morpholinyl-2 méthoxy)-8 tétrahydro-1,2,3,4 quinoléine et ses énantiomères.

6. La R,S trifluorométhyl-5 (morpholinyl-2 méthoxy)-8 tétrahydro-1,2,3,4 quinoléine et ses énantiomères.

7. La R,S fluoro-6 méthyl-1 (morpholinyl-2 méthoxy)-8 tétrahydro-1,2,3,4 quinoléine et ses énantiomères.

8. La R,S fluoro-5 méthyl-1 (morpholinyl-2 méthoxy)-8 tétrahydro-1,2,3,4 quinoléine et ses énantiomères.

9. La R,S trifluorométhyl-5 méthyl-1 (morpholinyl-2 méthoxy)-8 tétrahydro-1,2,3,4 quinoléine et ses énantiomères.

**10.** Le procédé de préparation des composés de la revendication 1 répondant à la formule générale I' :

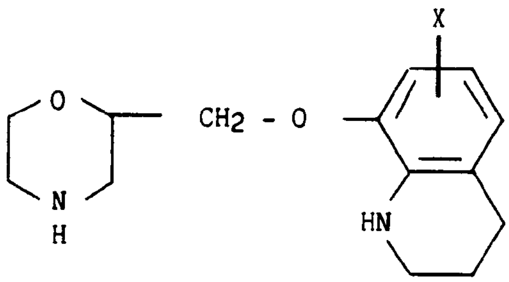

(I')

dans laquelle X a la signification définie dans la revendication 1,
caractérisé en ce que :

- l'on fait réagir une morpholine substituée de formule générale II :

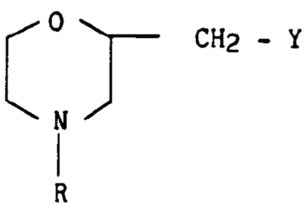

(II)

dans laquelle :

- R est un groupe protecteur tel qu'un groupe benzyle ou trityle et
- Y est un atome de chlore ou de brome ou un reste tosyloxy,

avec un sel alcalin de l'hydroxy-quinoléine de formule générale III :

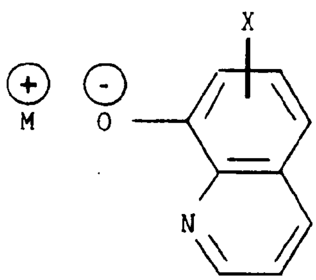

(III)

dans laquelle :

- X a la signification définie dans la revendication 1 et
- M est un métal alcalin tel que sodium ou potassium ;
- et l'on réduit le dérivé ainsi obtenu de formule générale IV :

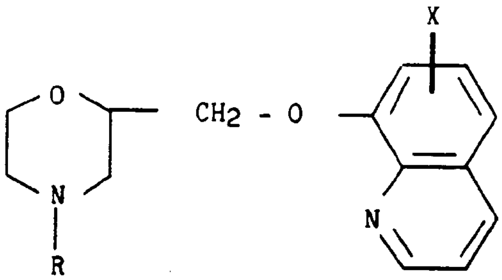

(IV)

dans laquelle R et X ont les significations précédemment définies.

**11.** Le procédé de préparation selon la revendication 10 caractérisé en ce que l'on effectue la condensation des dérivés II et III dans un solvant aprotique.

**12.** Le procédé de préparation selon la revendication 10 caractérisé en ce que l'on effectue la condensation du dérivé II avec le dérivé III à l'état de sel de sodium ou de potassium, par transfert de phase solide-liquide dans un solvant aprotique, en présence d'un sel d'ammonium quaternaire à haut poids moléculaire, à une température comprise entre 90 et 120°C.

**13.** Le procédé de préparation selon les revendications 10 à 12, caractérisé en ce que l'on effectue la réduction du dérivé IV, dans le cas où R est un radical benzyle, au moyen d'hydrogène à basse pression, en présence d'un catalyseur appartenant aux métaux du VIIIème groupe dans un solvant polaire, à une température comprise entre 20 et 60°C.

**14.** Le procédé de préparation selon les revendications 10 à 12 caractérisé en ce que la réduction s'effectue à partir d'un dérivé de formule IV dans laquelle R représente un radical trityle lequel par hydrolyse ménagée au moyen d'un acide donne un dérivé de formule IV dans laquelle R est remplacé par un atome d'hydrogène, et l'on réduit ce dernier par un borohydrure, dans un solvant polaire, à un pH compris entre 6 et 7.

**15.** Le procédé de préparation des composés de la revendication 1 répondant à la formule générale I" :

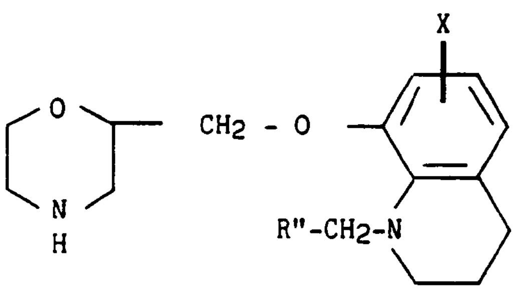

(I")

dans laquelle :
- X a la signification définie dans la revendication 1, et
- R" représente :
  . un atome d'hydrogène,
  . un radical alkyle ayant de 1 à 4 atomes de carbone en chaîne droite ou ramifiée comportant éventuellement une double liaison, ou
  . un radical aralkyle dans lequel la partie alkyle renferme de 1 à 4 atomes de carbone,

caractérisé en ce que :

- l'on réduit, le composé de formule IV défini dans la revendication 10, pour obtenir le composé de formule générale V :

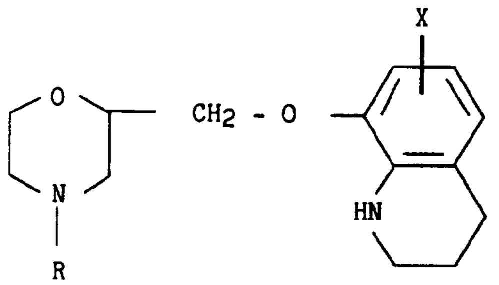

(V)

dans laquelle X et R ont les significations définies dans la revendication 1

- l'on alkyle ce composé V
  . soit au moyen d'un aldéhyde de formule : R"-CHO dans laquelle R" a la signification précédemment définie, et de $NaBH_3CN$, dans un solvant aprotique polaire à pH contrôlé ;
  . soit au moyen d'un acide de formule : R"-COOH dans laquelle R" a la signification définie ci-dessus, et de $NaBH_4$ dans un solvant aprotique polaire, et
- l'on débenzyle ou l'on détrityle le dérivé ainsi obtenu de formule générale VI :

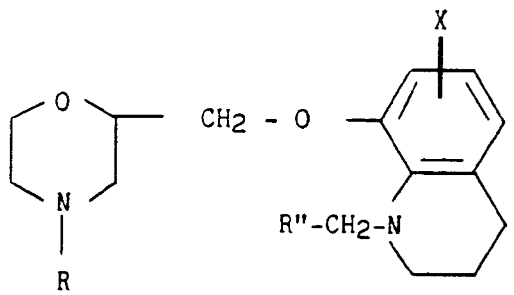

(VI)

dans laquelle X, R et R" ont les significations précédemment définies.

**16.** Les compositions pharmaceutiques contenant comme principe actif un composé selon les revendications 1 à 9, avec des excipients pharmaceutiques appropriés.

**17.** Les compositions pharmaceutiques selon la revendication 16, présentées sous une forme convenant notamment pour le traitement des syndromes ischémiques et du vieillissement cérébral.

**Revendications pour l'Etat contractant suivant : ES**

**1.** Le procédé de préparation des dérivés de la morpholine de formule générale (I) :

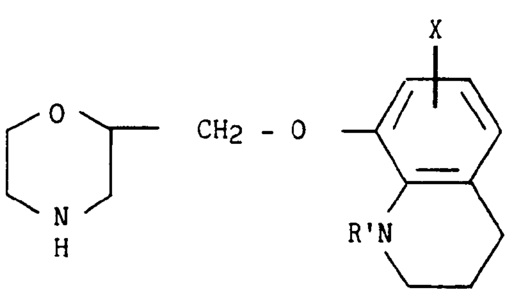

(I)

dans laquelle :

- X, fixé sur le noyau aromatique, représente :
  - un atome d'halogène tel qu'un atome de fluor ou de chlore, ou
  - un radical trifluorométhyle ; et

- R' représente :
  - un atome d'hydrogène,
  - un radical alkyle contenant de 1 à 5 atomes de carbone en chaîne droite ou ramifiée comportant éventuellement une double liaison, ou
  - un radical aralkyle dans lequel la partie alkyle renferme de 1 à 5 atomes de carbone ;
  
  sous formes racémique et énantiomères ;
  
  et de leurs sels physiologiquement tolérables avec des acides appropriés ;

caractérisé en ce que :

A) l'on fait réagir une morpholine substituée de formule générale II :

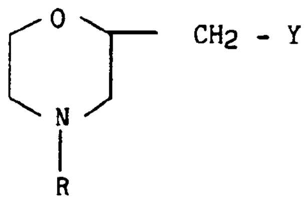

(II)

dans laquelle :

- R est un groupe protecteur tel qu'un groupe benzyle ou trityle et
- Y est un atome de chlore ou de brome ou un reste tosyloxy,

avec un sel alcalin de l'hydroxy-quinoléine de formule générale III :

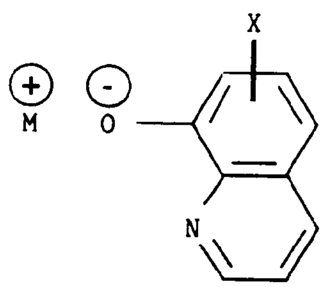

(III)

dans laquelle :

- X a la signification définie précédemment et
- M est un métal alcalin tel que sodium ou potassium ;
- et l'on réduit le dérivé ainsi obtenu de formule générale IV :

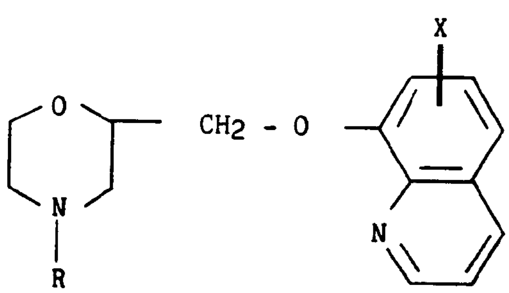

(IV)

dans laquelle R et X ont les significations précédemment définies ;

afin d'obtenir les dérivés I dans le cas où R' est un atome d'hydrogène, soit les dérivés répondant plus précisément à la formule générale I' :

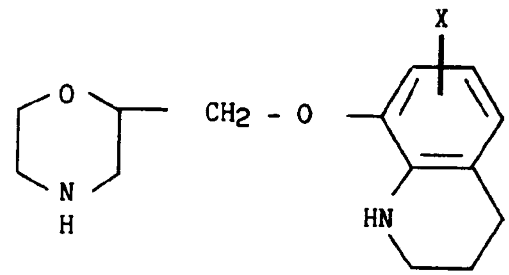

(I')

dans laquelle X a la signification précédemment définie ; et

- B) l'on réduit, le composé de formule IV défini précédemment, pour obtenir le composé de formule générale V :

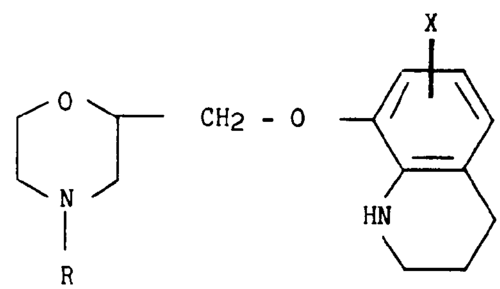

(V)

dans laquelle X et R ont les significations définies précédemment

- l'on alkyle ce composé V
  . soit au moyen d'un aldéhyde de formule : R"-CHO dans laquelle R" représente : un atome d'hydrogène,
  . un radical alkyle ayant de 1 à 4 atomes de carbone en chaîne droite ou ramifiée comportant éventuellement une double liaison, ou
  . un radical aralkyle dans lequel la partie alkyle renferme de 1 à 4 atomes de carbone, et de $NaBH_3CN$, dans un solvant aprotique polaire à pH contrôlé ;
  . soit au moyen d'un acide de formule : R"-COOH dans laquelle R" a la signification définie ci-dessus, et de $NaBH_4$ dans un solvant aprotique polaire, et
- l'on débenzyle ou l'on détrityle le dérivé ainsi obtenu de formule générale VI :

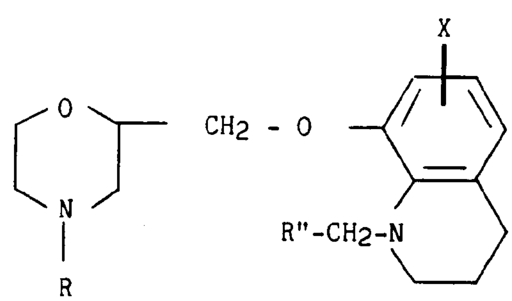

(VI)

dans laquelle X, R et R" ont les significations précédemment définies ; afin d'obtenir les dérivés I dans le cas où R' représente :

- un radical alkyle contenant de 1 à 5 atomes de carbone en chaîne droite ou ramifiée comportant éventuellement une double liaison, ou
- un radical aralkyle dans lequel la partie alkyle renferme de 1 à 5 atomes de carbone,

soit, les dérivés répondant plus précisément à la formule générale I" :

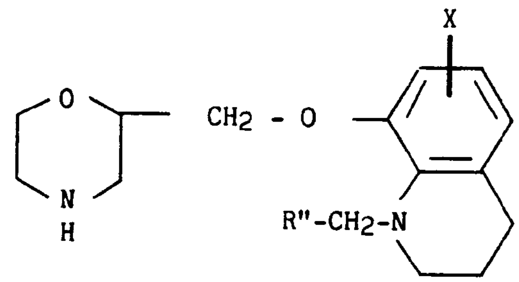

(I")

dans laquelle X et R" ont les significations précédemment définies.

- et si on le désire, on traite les dérivés I' et I" précédemment obtenus avec des acides appropriés pour donner les sels d'addition acides correspondants.

**2.** Le procédé de préparation selon la revendication 1, caractérisé en ce que l'on effectue la condensation des dérivés II et III dans un solvant aprotique.

**3.** Le procédé de préparation selon la revendication 1 caractérisé en ce que l'on effectue la condensation du dérivé II avec le dérivé III à l'état de sel de sodium ou de potassium, par transfert de phase solide-liquide dans un solvant aprotique, en présence d'un ssel d'ammonium quaternaire à haut poids moléculaire, à une température comprise entre 90 et 120°C.

**4.** Le procédé de préparation selon les revendications 1 à 3, caractérisé en ce que l'on effectue la réduction du dérivé IV, dans le cas où R est un radical benzyle, au moyen d'hydrogène à basse pression, en présence d'un catalyseur appartenant aux métaux du VIIIème groupe dans un solvant polaire, à une température comprise entre 20 et 60°C.

**5.** Le procédé de préparation selon les revendications 1 à 3, caractérisé en ce que la réduction s'effectue à partir d'un dérivé de formule IV dans laquelle R représente un radical trityle lequel par hydrolyse ménagée au moyen d'un acide donne un dérivé de formule IV dans laquelle R est remplacé par un atome d'hydrogène, et l'on réduit ce dernier par un borohydrure, dans un solvant polaire, à un pH compris entre 6 et 7.

**Revendications pour l'Etat contractant suivant : GR**

**1.** Le procédé de préparation des dérivés de la morpholine de formule générale (I) :

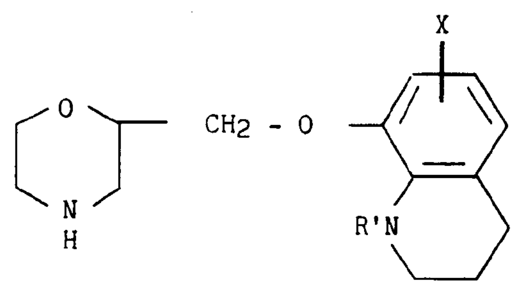

(I)

dans laquelle :

- X, fixé sur le noyau aromatique, représente :
  - un atome d'halogène tel qu'un atome de fluor ou de chlore, ou
  - un radical trifluorométhyle ; et
- R' représente :
  - un atome d'hydrogène,
  - un radical alkyle contenant de 1 à 5 atomes de carbone en chaîne droite ou ramifiée comportant éventuellement une double liaison, ou
  - un radical aralkyle dans lequel la partie alkyle renferme de 1 à 5 atomes de carbone ;
  sous formes racémique et énantiomères ;
  et de leurs sels physiologiquement tolérables avec des acides appropriés ;

caractérisé en ce que :

A) l'on fait réagir une morpholine substituée de formule générale II :

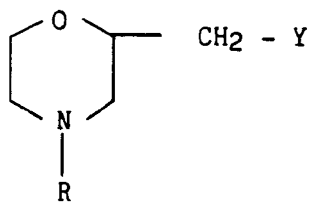

(II)

dans laquelle :

- R est un groupe protecteur tel qu'un groupe benzyle ou trityle et
- Y est un atome de chlore ou de brome ou un reste tosyloxy,

avec un sel alcalin de l'hydroxy-quinoléine de formule générale III :

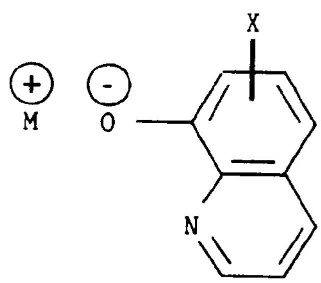

(III)

dans laquelle :

- X a la signification définie précédemment et
- M est un métal alcalin tel que sodium ou potassium ;
- et l'on réduit le dérivé ainsi obtenu de formule générale IV :

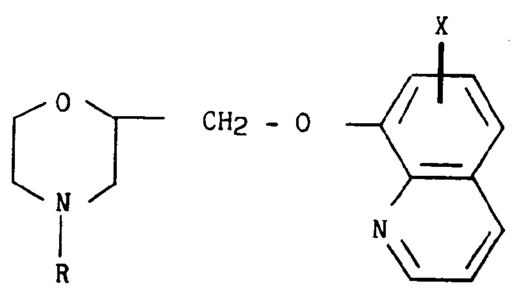

(IV)

dans laquelle R et X ont les significations précédemment définies ;

afin d'obtenir les dérivés I dans le cas où R' est un atome d'hydrogène, soit les dérivés répondant plus précisément à la formule générale I' :

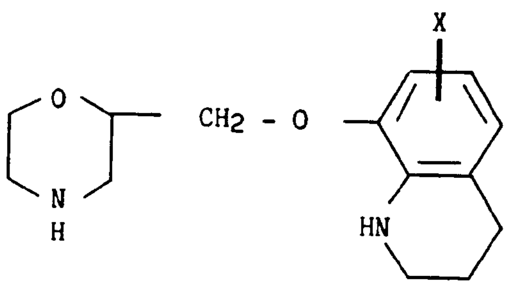

(I')

dans laquelle X a la signification précédemment définie ; et

- B) l'on réduit, le composé de formule IV défini précédemment, pour obtenir le composé de formule générale V :

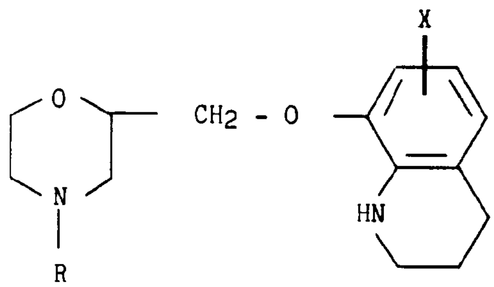

(V)

dans laquelle X et R ont les significations définies précédemment

- l'on alkyle ce composé V
  . soit au moyen d'un aldéhyde de formule : R"-CHO dans laquelle R" représente :
  un atome d'hydrogène,
  . un radical alkyle ayant de 1 à 4 atomes de carbone en chaîne droite ou ramifiée comportant éventuellement une double liaison, ou
  . un radical aralkyle dans lequel la partie alkyle renferme de 1 à 4 atomes de carbone,
  et de $NaBH_3CN$, dans un solvant aprotique polaire à pH contrôlé ;
  . soit au moyen d'un acide de formule : R"-COOH dans laquelle R" a la signification définie ci-dessus, et de $NaBH_4$ dans un solvant aprotique polaire, et
- l'on débenzyle ou l'on détrityle le dérivé ainsi obtenu de formule générale VI :

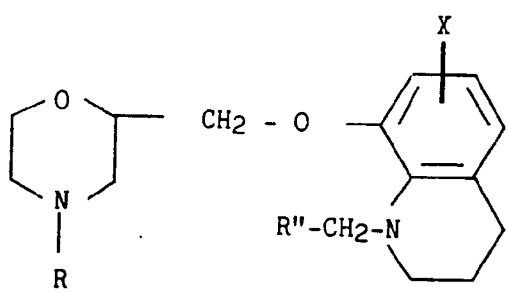

(VI)

dans laquelle X, R et R" ont les significations précédemment définies afin d'obtenir les dérivés I dans le cas où R' représente :

- un radical alkyle contenant de 1 à 5 atomes de carbone en chaîne droite ou ramifiée comportant éventuellement une double liaison, ou
- un radical aralkyle dans lequel la partie alkyle renferme de 1 à 5 atomes de carbone,

soit, les dérivés répondant plus précisément à la formule générale I" :

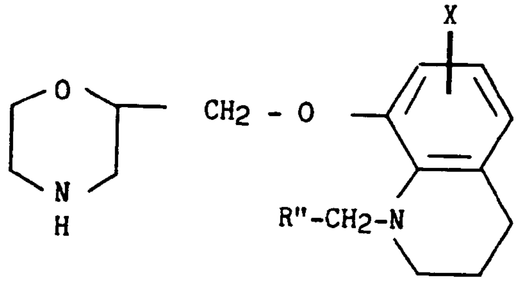

(I")

dans laquelle X et R" ont les significations précédemment définies.

- et si on le désire, on traite les dérivés I' et I" précédemment obtenus avec des acides appropriés

pour donner les sels d'addition acides correspondants.

2. Le procédé de préparation selon la revendication 1, caractérisé en ce que l'on effectue la condensation des dérivés II et III dans un solvant aprotique.

3. Le procédé de préparation selon la revendication 1 caractérisé en ce que l'on effectue la condensation du dérivé II avec le dérivé III à l'état de sel de sodium ou de potassium, par transfert de phase solide-liquide dans un solvant aprotique, en présence d'un sel d'ammonium quaternaire à haut poids moléculaire, à une température comprise entre 90 et 120°C.

4. Le procédé de préparation selon les revendications 1 à 3, caractérisé en ce que l'on effectue la réduction du dérivé IV, dans le cas où R est un radical benzyle, au moyen d'hydrogène à basse pression, en présence d'un catalyseur appartenant aux métaux du VIIIème groupe dans un solvant polaire, à une température comprise entre 20 et 60°C.

5. Le procédé de préparation selon les revendications 1 à 3 caractérisé en ce que la réduction s'effectue à partir d'un dérivé de formule IV dans laquelle R représente un radical trityle lequel par hydrolyse ménagée au moyen d'un acide donne un dérivé de formule IV dans laquelle R est remplacé par un atome d'hydrogène, et l'on réduit ce dernier par un borohydrure, dans un solvant polaire, à un pH compris entre 6 et 7.

## Patentansprüche

### Patentansprüche für folgende Vertragsstaaten : AT, BE, CH, DE, DK, FR, GB, IT, LI, LU, NL, SE

1. Morpholinderivate der allgemeinen Formel (I):

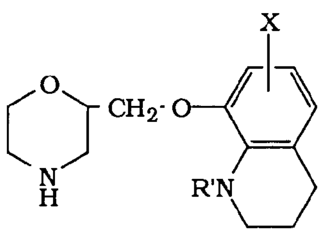

(I)

in der

- X, das an den aromatischen Kern gebunden ist,
  - ein Halogenatom, wie ein Fluor- oder Chloratom oder
  - eine Trifluormethylgruppe; und
- R'
  - ein Wasserstoffatom,
  - eine Alkylgruppe mit 1 bis 5 Kohlenstoffatomen in gerader oder verzweigter Kette, die gegebenenfalls eine Doppelbindung aufweist, oder
  - eine Aralkylgruppe, deren Alkylteil 1 bis 5 Kohlenstoffatome umfaßt; in Form des Racemats und der Enantiomeren.

2. Physiologisch verträgliche Salze der Verbindungen nach Anspruch 1 mit geeigneten Säuren.

3. R,S-6-Fluor-8-(morpholin-2-yl-methoxy)-1,2,3,4-tetrahydro-chinolin und dessen Enantiomere.

4. R,S-5-Fluor-8-(morpholin-2-yl-methoxy)-1,2,3,4-tetrahydro-chinolin und dessen Enantiomere.

5. R,S-5-Chlor-8-(morpholin-2-yl-methoxy)-1,2,3,4-tetrahydro-chinolin und dessen Enantiomere.

6. R,S-5-Trifluormethyl-8-(morpholin-2-yl-methoxy)-1,2,3 ,4-tetrahydrochinolin und dessen Enantiomere.

7. R,S-6-Fluor-1-methyl-8-(morpholin-2-yl-methoxy)-1,2,3,4-tetrahydrochinolin und dessen Enantiomere.

**8.** R,S-5-Fluor-1-methyl-8-(morpholin-2-yl-methoxy)- 1,2,3,4-tetrahydrochinolin und dessen Enantiomere.

**9.** R,S-5-Trifluormethyl-1-methyl-8-(morpholin-2-yl-methoxy)-1,2,3,4-tetrahydro-chinolin und dessen Enantiomere.

**10.** Verfahren zur Herstellung der Verbindungen nach Anspruch 1 der allgemeinen Formel I':

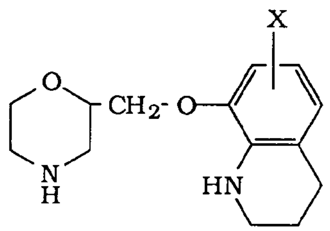

(I')

in der X die in Anspruch 1 angegebenen Bedeutungen besitzt, **dadurch gekennzeichnet,** daß man

- ein substituiertes Morpholin der allgemeinen Formel II:

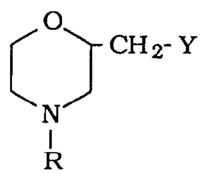

(II)

in der

- R eine Schutzgruppe, wie eine Benzylgruppe oder eine Tritylgruppe und
- Y ein Chlor- oder Bromatom oder eine Tosyloxygruppe bedeuten, mit einem Alkalimetallsalz des Hydroxychinolins der allgemeinen Formel III:

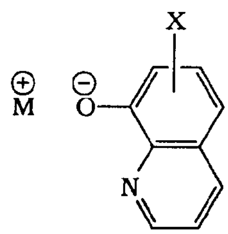

(III)

in der

- X die in Anspruch 1 angegebenen Bedeutungen besitzt und
- M ein Alkalimetall, wie Natrium oder Kalium, bedeutet; umsetzt
- und das in dieser Weise erhaltene Derivat der allgemeinen Formel IV:

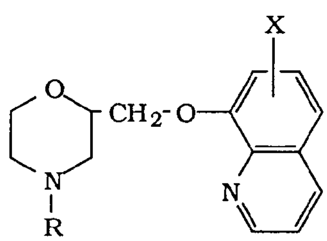

(IV)

in der R und X die oben angegebenen Bedeutungen besitzen, reduziert.

**11.** Verfahren nach Anspruch 10, **dadurch gekennzeichnet,** daß man die Kondensation der Derivate II und

III in einem aprotischen Lösungsmittel durchführt.

**12.** Verfahren nach Anspruch 10, **dadurch gekennzeichnet,** daß man die Kondensation des Derivats II mit dem Derivat III in Form des Natrium- oder Kaliumsalzes durch Fest-Flüssig-Phasen-Transfer in einem aprotischen Lösungsmittel in Gegenwart eines quaternären Ammoniumsalzes mit hohem Molekulargewicht bei einer Temperatur zwischen 90 und 120°C bewirkt.

**13.** Verfahren nach den Ansprüchen 10 bis 12, **dadurch gekennzeichnet,** daß man die Reduktion des Derivats IV dann, wenn R eine Benzylgruppe darstellt, mit Wasserstoff bei niedrigem Druck in Gegenwart eines Katalysators. der zu den Metallen der Gruppe VIII des Periodensystems gehört, in einem polaren Lösungsmittel bei einer Temperatur zwischen 20 und 60°C bewirkt.

**14.** Verfahren nach den Ansprüchen 10 bis 12, **dadurch gekennzeichnet,** daß die Reduktion ausgehend von einem Derivat der Formel IV, in der R eine Tritylgruppe bedeutet, erfolgt, welches durch milde Hydrolyse mit Hilfe einer Säure ein Derivat der Formel IV ergibt, in der R durch ein Wasserstoffatom ersetzt ist, und man dieses letztere mit einem Boranat in einem polaren Lösungsmittel bei einem pH-Wert zwischen 6 und 7 reduziert.

**15.** Verfahren zur Herstellung der Verbindungen nach Anspruch 1 der allgemeinen Formel I"

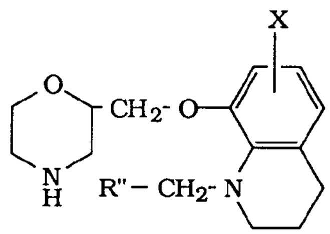

(I")

in der
- X die in Anspruch 1 angegebenen Bedeutungen besitzt und
- R"
  . ein Wasserstoffatom,
  . eine Alkylgruppe mit 1 bis 4 Kohlenstoffatomen in gerader oder verzweigter Kette, die gegebenenfalls eine Doppelbindung aufweist, oder
  . eine Aralkylgruppe, deren Alkylteil 1 bis 4 Kohlenstoffatome enthält, bedeutet,

**dadurch gekennzeichnet,** daß man
- die in Anspruch 10 definierte Verbindung der Formel IV reduziert zur Bildung der Verbindung der allgemeinen Formel V:

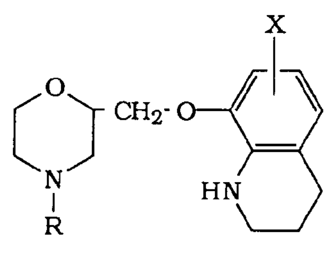

(V)

in der X und R die in Anspruch 1 angegebenen Bedeutungen besitzen,
- die Verbindung V
  . entweder mit einem Aldehyd der Formel R"-CHO, in der R" die oben angegebenen Bedeutungen besitzt, und $NaBH_3CN$ in einem polaren aprotischen Lösungsmittel mit gesteuertem pH-Wert;
  . oder mit einer Säure der Formel R"-COOH, in der R" die oben angegebenen Bedeutungen besitzt, und $NaBH_4$ in einem polaren aprotischen Lösungsmittel alkyliert und
- das in dieser Weise erhaltene Derivat der allgemeinen Formel VI:

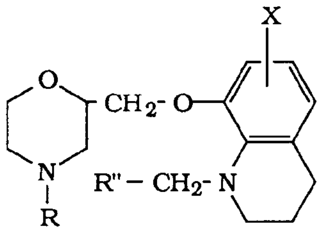

(VI)

in der X, R und R” die oben angegebenen Bedeutungen besitzen, debenzyliert oder detrityliert.

**16.** Pharmazeutische Zubereitungen enthaltend als Wirkstoff eine Verbindung nach einem der Ansprüche 1 bis 9 zusammen mit geeigneten pharmazeutischen Trägermaterialien.

**17.** Pharmazeutische Zubereitungen nach Anspruch 16 in einer insbesondere zur Behandlung von ischämischen Syndromen und des zerebralen Alterns geeigneten Form.

**Patentansprüche für folgenden Vertragsstaat : ES**

**1.** Verfahren zur Herstellung von Morpholinderivaten der allgemeinen Formel (I):

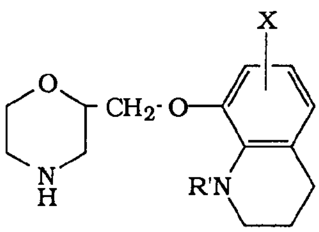

(I)

in der

- X, das an den aromatischen Kern gebunden ist.
  - ein Halogenatom, wie ein Fluor- oder Chloratom oder
  - eine Trifluormethylgruppe; und
- R’
  - ein Wasserstoffatom,
  - eine Alkylgruppe mit 1 bis 5 Kohlenstoffatomen in gerader oder verzweigter Kette, die gegebenenfalls eine Doppelbindung aufweist, oder
  - eine Aralkylgruppe, deren Alkylteil 1 bis 5 Kohlenstoffatome umfaßt; in Form des Racemats und der Enantiomeren und von deren physiologisch verträglichen Salzen mit geeigneten Säuren, **dadurch gekennzeichnet,** daß man

A) ein substituiertes Morpholin der allgemeinen Formel II:

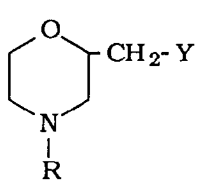

(II)

in der

- R eine Schutzgruppe, wie eine Benzylgruppe oder eine Tritylgruppe und
- Y ein Chlor- oder Bromatom oder eine Tosyloxygruppe bedeuten, mit einem Alkalimetallsalz des Hydroxychinolins der allgemeinen Formel III:

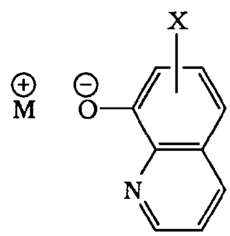

(III)

in der

- X die in Anspruch 1 angegebenen Bedeutungen besitzt und
- M ein Alkalimetall, wie Natrium oder Kalium, bedeutet; umsetzt
- und das in dieser Weise erhaltene Derivat der allgemeinen Formel IV:

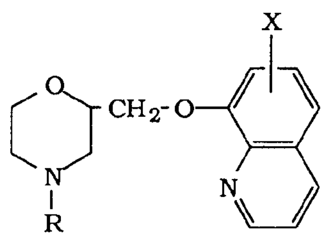

(IV)

in der R und X die oben angegebenen Bedeutungen besitzen, reduziert; so daß man die Derivate I erhält, worin R' ein Wasserstoffatom bedeutet, d. h. genauer die Derivate der allgemeinen Formel I':

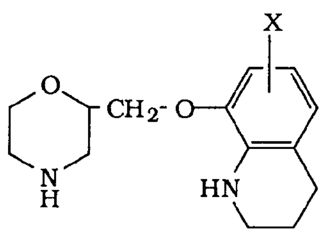

(I')

in der X die oben angegebenen Bedeutungen besitzt; und

- B) die oben definierte Verbindung der Formel IV reduziert, so daß man die Verbindung der allgemeinen Formel V:

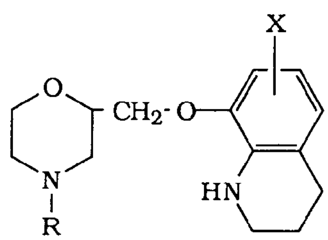

(V)

erhält, in der X und R die oben angegebenen Bedeutungen besitzen,

- welche Verbindung V man
  . entweder mit Hilfe eines Aldehyds der Formel R"-CHO, worin R" ein Wasserstoffatom,
  . eine Alkylgruppe mit 1 bis 4 Kohlenstoffatomen in gerader oder verzweigter Kette, die gegebenenfalls eine Doppelbindung aufweist, oder
  . eine Aralkylgruppe, in der der Alkylteil 1 bis 4 Kohlenstoffatome umfaßt, bedeutet, und $NaBH_3CN$ in einem aprotischen polaren Lösungsmittel mit gesteuertem pH-Wert;
  . oder mit einer Säure der Formel R"-COOH, in der R" die oben angegebenen Bedeutungen besitzt, und $NaBH_4$ in einem aprotischen polaren Lösungsmittel

  alkyliert und

- das in dieser Weise erhaltene Derivat der allgemeinen Formel VI

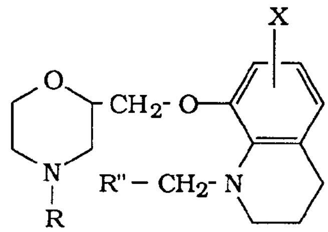

(VI)

in der X, R und R" die oben angegebenen Bedeutungen besitzen, debenzyliert oder detrityliert, so daß man die Derivate I erhält, worin R':
- eine Alkylgruppe mit 1 bis 5 Kohlenstoffatomen in gerader oder verzweigter Kette, die gegebenenfalls eine Doppelbindung aufweist, oder
- eine Aralkylgruppe, deren Alkylteil 1 bis 5 Kohlenstoffatome aufweist, bedeutet,

d. h. Derivate, die genauer der allgemeinen Formel I":

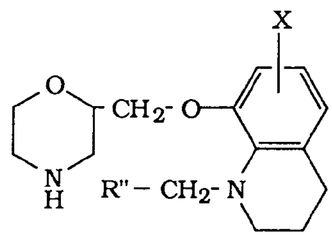

(I")

entsprechen, in der X und R" die oben angegebenen Bedeutungen besitzen,

- und gewünschtenfalls die erhaltenen Derivate I' und I" mit geeigneten Säuren behandelt zur Bildung der entsprechenden Säureadditionssalze.

**2.** Verfahren nach Anspruch 1, **dadurch gekennzeichnet,** daß man die Kondensation der Derivate II und III in einem aprotischen Lösungsmittel bewirkt.

**3.** Verfahren nach Anspruch 1, **dadurch gekennzeichnet,** daß daß man die Kondensation des Derivats II mit dem Derivat III in Form des Natrium- oder Kaliumsalzes durch Fest-Flüssig-Phasen-Transfer in einem aprotischen Lösungsmittel in Gegenwart eines quaternären Ammoniumsalzes mit hohem Molekulargewicht bei einer Temperatur zwischen 90 und 120°C bewirkt.

**4.** Verfahren nach den Ansprüchen 1 bis 3, dadurch gekennzeichnet, daß man die Reduktion des Derivats IV dann, wenn R eine Benzylgruppe darstellt, mit Wasserstoff bei niedrigem Druck in Gegenwart eines Katalysators, der zu den Metallen der Gruppe VIII des Periodensystems gehört, in einem polaren Lösungsmittel bei einer Temperatur zwischen 20 und 60°C bewirkt.

**5.** Verfahren nach den Ansprüchen 1 bis 3, **dadurch gekennzeichnet,** daß die Reduktion ausgehend von einem Derivat der Formel IV, in der R eine Tritylgruppe bedeutet, erfolgt, welches durch milde Hydrolyse mit Hilfe einer Säure ein Derivat der Formel IV ergibt, in der R durch ein Wasserstoffatom ersetzt ist, und man dieses letztere mit einem Boranat in einem polaren Lösungsmittel bei einem pH-Wert zwischen 6 und 7 reduziert.

**Patentansprüche für folgenden Vertragsstaat : GR**

**1.** Verfahren zur Herstellung von Morpholinderivaten der allgemeinen Formel (I):

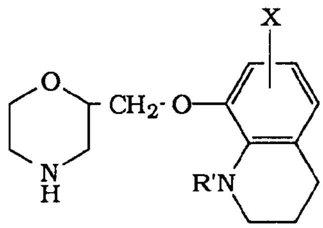

(I)

in der

- X, das an den aromatischen Kern gebunden ist,
  - ein Halogenatom, wie ein Fluor- oder Chloratom oder
  - eine Trifluormethylgruppe; und
- R'
  - ein Wasserstoffatom,
  - eine Alkylgruppe mit 1 bis 5 Kohlenstoffatomen in gerader oder verzweigter Kette, die gegebenenfalls eine Doppelbindung aufweist, oder
  - eine Aralkylgruppe, deren Alkylteil 1 bis 5 Kohlenstoffatome umfaßt;

in Form des Racemats und der Enantiomeren und von deren physiologisch verträglichen Salzen mit geeigneten Säuren, **dadurch gekennzeichnet,** daß man

A) ein substituiertes Morpholin der allgemeinen Formel II:

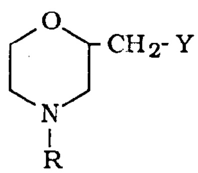

(II)

in der

- R eine Schutzgruppe, wie eine Benzylgruppe oder eine Tritylgruppe und
- Y ein Chlor- oder Bromatom oder eine Tosyloxygruppe bedeuten, mit einem Alkalimetallsalz des Hydroxychinolins der allgemeinen Formel III:

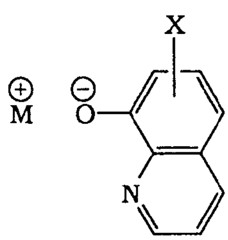

(III)

in der

- X die in Anspruch 1 angegebenen Bedeutungen besitzt und
- M ein Alkalimetall, wie Natrium oder Kalium, bedeutet; umsetzt
- und das in dieser Weise erhaltene Derivat der allgemeinen Formel IV:

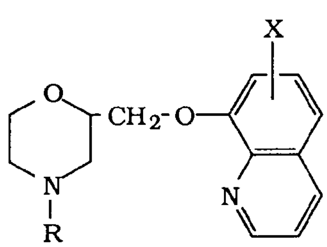

(IV)

in der R und X die oben angegebenen Bedeutungen besitzen, reduziert; so daß man die Derivate

I erhält, worin R' ein Wasserstoffatom bedeutet, d. h. genauer die Derivate der allgemeinen Formel I':

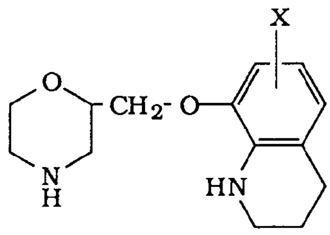

(I')

in der X die oben angegebenen Bedeutungen besitzt; und

- B) die oben definierte Verbindung der Formel IV reduziert, so daß man die Verbindung der allgemeinen Formel V:

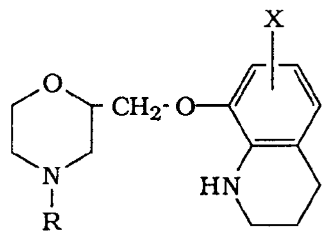

(V)

erhält, in der X und R die oben angegebenen Bedeutungen besitzen,

- welche Verbindung V man
  . entweder mit Hilfe eines Aldehyds der Formel R"-CHO, worin R" ein Wasserstoffatom,
  . eine Alkylgruppe mit 1 bis 4 Kohlenstoffatomen in gerader oder verzweigter Kette, die gegebenenfalls eine Doppelbindung aufweist, oder
  . eine Aralkylgruppe, in der der Alkylteil 1 bis 4 Kohlenstoffatome umfaßt, bedeutet, und $NaBH_3CN$ in einem aprotischen polaren Lösungsmittel mit gesteuertem pH-Wert;
  . oder mit einer Säure der Formel R"-COOH, in der R" die oben angegebenen Bedeutungen besitzt, und $NaBH_4$ in einem aprotischen polaren Lösungsmittel

  alkyliert und
- das in dieser Weise erhaltene Derivat der allgemeinen Formel VI

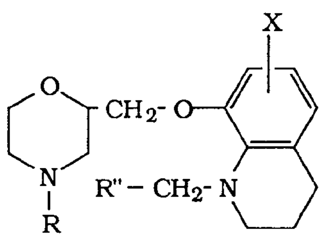

(VI)

in der X, R und R" die oben angegebenen Bedeutungen besitzen, debenzyliert oder detrityliert, so daß man die Derivate I erhält, worin R':

- eine Alkylgruppe mit 1 bis 5 Kohlenstoffatomen in gerader oder verzweigter Kette, die gegebenenfalls eine Doppelbindung aufweist, oder
- eine Aralkylgruppe, deren Alkylteil 1 bis 5 Kohlenstoffatome aufweist, bedeutet,

d. h. Derivate, die genauer der allgemeinen Formel I":

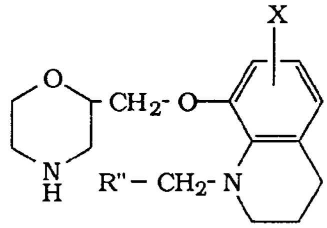

(I")

entsprechen, in der X und R" die oben angegebenen Bedeutungen besitzen,

- und gewünschtenfalls die erhaltenen Derivate I' und I" mit geeigneten Säuren behandelt zur Bildung der entsprechenden Säureadditionssalze.

2. Verfahren nach Anspruch 1, **dadurch gekennzeichnet,** daß man die Kondensation der Derivate II und III in einem aprotischen Lösungsmittel bewirkt.

3. Verfahren nach Anspruch 1, **dadurch gekennzeichnet,** daß daß man die Kondensation des Derivats II mit dem Derivat III in Form des Natrium- oder Kaliumsalzes durch Fest-Flüssig-Phasen-Transfer in einem aprotischen Lösungsmittel in Gegenwart eines quaternären Ammoniumsalzes mit hohem Molekulargewicht bei einer Temperatur zwischen 90 und 120°C bewirkt.

4. Verfahren nach den Ansprüchen 1 bis 3, **dadurch gekennzeichnet,** daß man die Reduktion des Derivats IV dann, wenn R eine Benzylgruppe darstellt, mit Wasserstoff bei niedrigem Druck in Gegenwart eines Katalysators, der zu den Metallen der Gruppe VIII des Periodensystems gehört, in einem polaren Lösungsmittel bei einer Temperatur zwischen 20 und 60°C bewirkt.

5. Verfahren nach den Ansprüchen 1 bis 3, **dadurch gekennzeichnet,** daß die Reduktion ausgehend von einem Derivat der Formel IV, in der R eine Tritylgruppe bedeutet, erfolgt, welches durch milde Hydrolyse mit Hilfe einer Säure ein Derivat der Formel IV ergibt, in der R durch ein Wasserstoffatom ersetzt ist, und man dieses letztere mit einem Boranat in einem polaren Lösungsmittel bei einem pH-Wert zwischen 6 und 7 reduziert.

**Claims**

**Claims for the following Contracting States : AT, BE, CH, DE, DK, FR, GB, IT, LI, LU, NL, SE**

1. Morpholine compounds of the general formula (I):

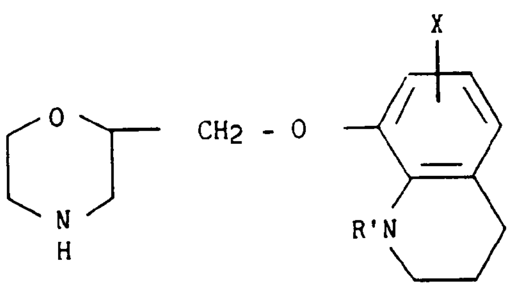

(I)

wherein :
- X, attached to the aromatic nucleus, represents :
  - a halogen atom, such as a fluorine or chlorine atom, or
  - a trifluoromethyl radical; and
- R' represents:
  - a hydrogen atom,
  - a straight-chain or branched alkyl radical containing from 1 to 5 carbon atoms and optionally containing a double bond, or

- an aralkyl radical in which the alkyl moiety contains from 1 to 5 carbon atoms;

in racemic form and in the form of enantiomers.

**2.** Physiologically tolerable salts of the compounds of claim 1 with appropriate acids.

**3.** R,S-6-fluoro-8-(2-morpholinylmethoxy)-1,2,3,4-tetrahydroquinoline and its enantiomers.

**4.** R,S-5-fluoro-8-(2-morpholinylmethoxy)-1,2,3,4-tetrahydroquinoline and its enantiomers.

**5.** R,S-5-chloro-8-(2-morpholinylmethoxy)-1,2,3,4-tetrahydroquinoline and its enantiomers.

**6.** R,S-5-trifluoromethyl-8-(2-morpholinylmethoxy)-1,2,3,4-tetrahydroquinoline and its enantiomers.

**7.** R,S-6-fluoro-1-methyl-8-(2-morpholinylmethoxy)-1,2,3,4-tetrahydroquinoline and its enantiomers.

**8.** R,S-5-fluoro-1-methyl-8-(2-morpholinylmethoxy)-1,2,3,4-tetrahydroquinoline and its enantiomers.

**9.** R,S-5-trifluoromethyl-1-methyl-8-(2-morpholinylmethoxy)-1,2,3,4-tetrahydroquinoline and its enantiomers.

**10.** A process for the preparation of compounds of claim 1 corresponding to the general formula I' :

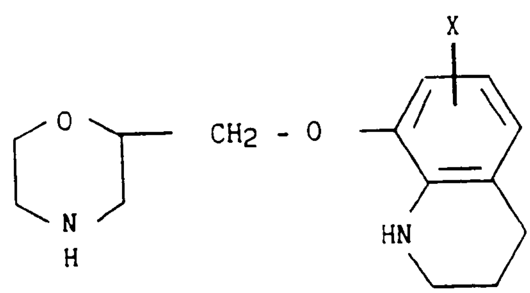

(I')

wherein X is as defined in claim 1,

characterised in that:

- a substituted morpholine of the general formula II :

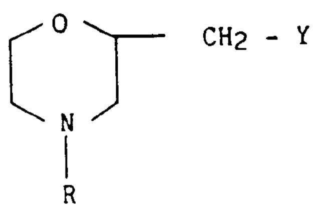

(II)

wherein :

- R is a protecting group, such as a benzyl or trityl group and
- Y is a chlorine or bromine atom or a tosyloxy radical,

is reacted with an alkali metal salt of hydroxyquinoline of the general formula III :

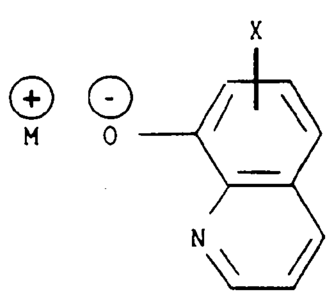

(III)

wherein :

- X is as defined in claim 1 and
- M is an alkali metal, such as sodium or potassium;
- and the compound so obtained of the general formula IV :

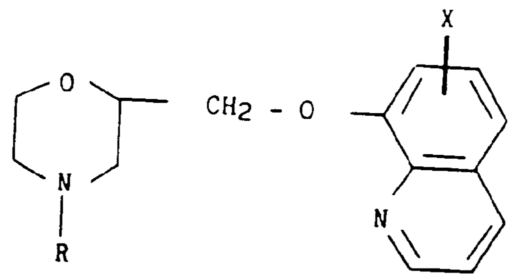

(IV)

wherein R and X are as defined hereinbefore, is reduced.

**11.** A preparation process according to claim 10, characterised in that the condensation of compounds II and III is carried out in an aprotic solvent.

**12.** A preparation process according to claim 10. characterised in that the condensation of compound II with compound III in the form of the potassium or sodium salt is carried out by solid-liquid phase transfer in an aprotic solvent, in the presence of a quaternary ammonium salt having a high molecular weight, at a temperature of from 90 to 120°C.

**13.** A preparation process according to any one of claims 10 to 12, characterised in that the reduction of compound IV wherein R is a benzyl radical is carried out by means of hydrogen at reduced pressure, in the presence of a catalyst belonging to the metals of group VIII in a polar solvent, at a temperature of from 20 to 60°C.

**14.** A preparation process according to any one of claims 10 to 12, characterised in that the reduction is effected starting from a compound of formula IV wherein R represents a trityl radical which, by means of hydrolysis carried out using an acid, yields a compound of formula IV wherein R has been replaced by a hydrogen atom, and the latter compound is reduced by a borohydride, in a polar solvent, at a pH of from 6 to 7.

**15.** A process for the preparation of compounds of claim 1 corresponding to the general formula I" :

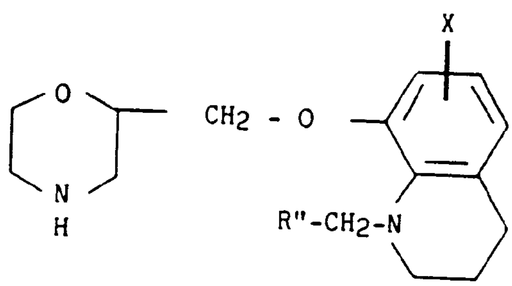

(I")

wherein :

- X is as defined in claim 1, and
- R" represents :
  . a hydrogen atom,
  . a straight-chain or branched alkyl radical containing from 1 to 4 carbon atoms and optionally containing a double bond, or
  . an aralkyl radical in which the alkyl moiety contains from 1 to 4 carbon atoms,

  characterised in that :
- the compound of formula IV defined in claim 10 is reduced to yield a compound of the general formula V :

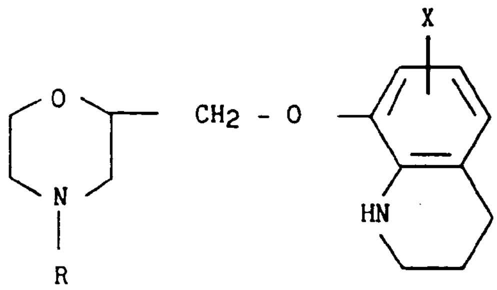

(V)

wherein X and R are as defined in claim 1,

- the compound V is alkylated
  - . either by means of an aldehyde of formula R"-CHO wherein R" is as defined hereinbefore, and $NaBH_3CN$, in a polar aprotic solvent with controlled pH;
  - . or by means of an acid of formula R"-COOH wherein R" is as defined above, and NaBH4 in a polar aprotic solvent, and
- the compound so obtained of the general formula VI :

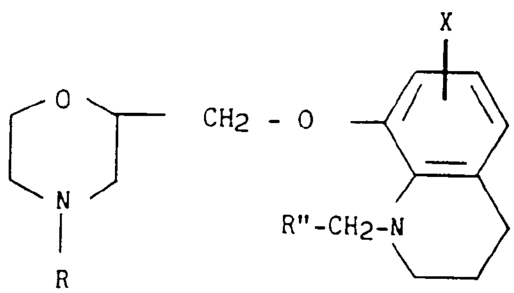

(VI)

wherein X, R and R" are as defined hereinbefore, is debenzylated or detritylated.

**16.** Pharmaceutical compositions comprising as active ingredient a compound according to any one of claims 1 to 9 together with appropriate pharmaceutical excipients.

**17.** Pharmaceutical compositions according to claim 16, presented in a form suitable especially for the treatment of ischaemic syndromes and cerebral ageing.

**Claims for the following Contracting State : ES**

**1.** A process for the preparation of morpholine compounds of the general formula (I) :

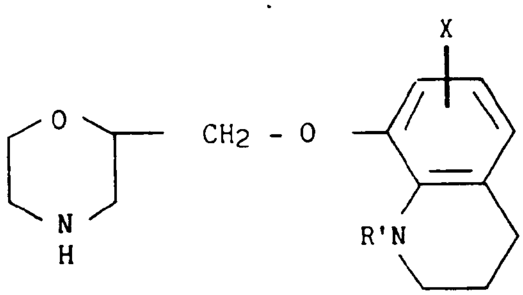

(I)

wherein :

- X, attached to the aromatic nucleus, represents :
  - a halogen atom, such as a fluorine or chlorine atom, or
  - a trifluoromethyl radical; and
- R' represents:

- a hydrogen atom,
- a straight-chain or branched alkyl radical containing from 1 to 5 carbon atoms and optionally containing a double bond, or
- an aralkyl radical in which the alkyl moiety contains from 1 to 5 carbon atoms;

in racemic form and in the form of enantiomers, and their physiologically tolerable salts with appropriate acids,

characterised in that :

A) a substituted morpholine of the general formula II :

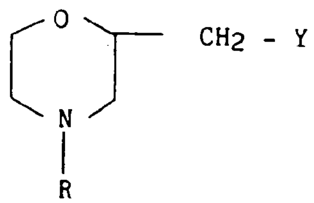

(II)

wherein :

- R is a protecting group, such as a benzyl or trityl group and
- Y is a chlorine or bromine atom or a tosyloxy radical,

is reacted with an alkali metal salt of hydroxyquinoline of the general formula III :

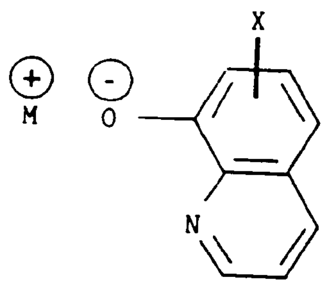

(III)

wherein :

- X is as defined hereinbefore and
- M is an alkali metal, such as sodium or potassium;
- and the compound so obtained of the general formula IV :

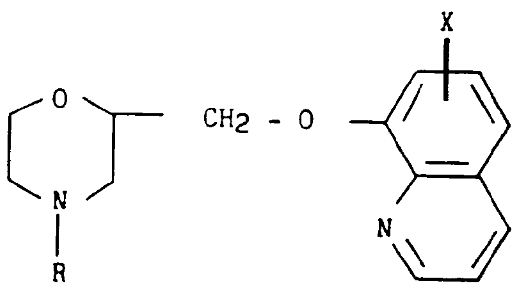

(IV)

wherein R and X are as defined hereinbefore, is reduced

in order to obtain compounds I wherein R' is a hydrogen atom, that is to say compounds corresponding more precisely to the general formula I'

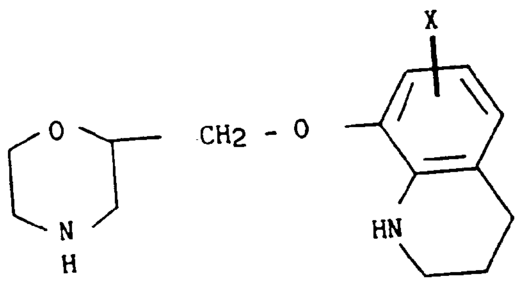

(I')

wherein X is as defined hereinbefore, or

-B) the compound of formula IV defined hereinbefore is reduced to obtain the compound of the general formula V :

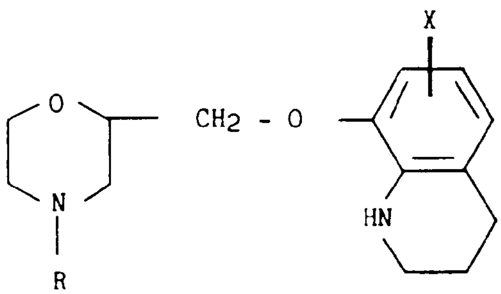

(V)

wherein X and R are as defined hereinbefore,

- the compound V is alkylated
  . either by means of an aldehyde of formula R"-CHO wherein R" represents:
  a hydrogen atom
  . a straight-chain or branched alkyl radical containing from 1 to 4 carbon atoms and optionally containing a double bond, or
  . an aralkyl radical in which the alkyl moiety contains from 1 to 4 carbon atoms,
  and by means of $NaBH_3CN$, in a polar aprotic solvent with controlled pH;
  . or by means of an acid of formula R"-COOH wherein R" is as defined above, and NaBH4 in a polar aprotic solvent, and
- the compound so obtained of the general formula VI :

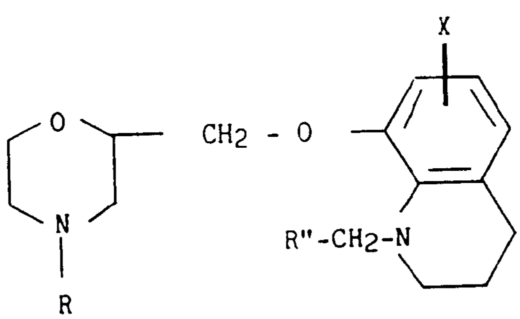

(VI)

wherein X, R and R" are as defined hereinbefore, is debenzylated or detritylated to obtain compounds I wherein R' represents :

- a straight-chain or branched alkyl radical containing from 1 to 5 carbon atoms and optionally containing a double bond, or
- an aralkyl radical in which the alkyl moiety contains from 1 to 5 carbon atoms,

that is to say, compounds corresponding more precisely to the general formula I" :

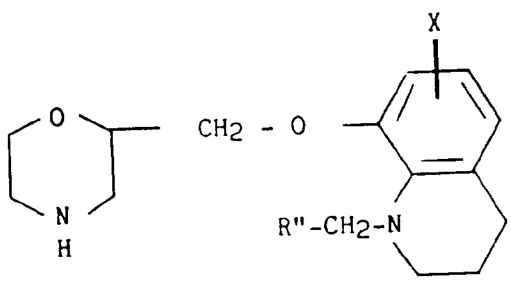

(I")

wherein X and R" are as defined hereinbefore,

- and, if desired, the compounds I' and I" obtained above are treated with appropriate acids to yield the corresponding acid addition salts.

2. A preparation process according to claim 1, characterised in that the condensation of compounds II and III is carried out in an aprotic solvent.

3. A preparation process according to claim 1, characterised in that the condensation of compound II with compound III in the form of the potassium or sodium salt is carried out by solid-liquid phase transfer in an aprotic solvent, in the presence of a quaternary ammonium salt having a high molecular weight, at a temperature of from 90 to 120°C.

4. A preparation process according to any one of claims 1 to 3, characterised in that the reduction of compound IV wherein R is a benzyl radical is carried out by means of hydrogen at reduced pressure, in the presence of a catalyst belonging to the metals of group VIII in a polar solvent, at a temperature of from 20 to 60°C.

5. A preparation process according to any one of claims 1 to 3, characterised in that the reduction is effected starting from a compound of formula IV wherein R represents a trityl radical which, by means of hydrolysis carried out using an acid, yields a compound of formula IV wherein R has been replaced by a hydrogen atom, and the latter compound is reduced by a borohydride, in a polar solvent, at a pH of from 6 to 7.

**Claims for the following Contracting State : GR**

1. A process for the preparation of morpholine compounds of the general formula (I) :

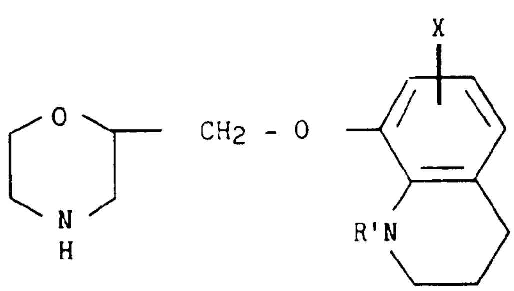

(I)

wherein :

- X, attached to the aromatic nucleus, represents :
  - a halogen atom, such as a fluorine or chlorine atom, or
  - a trifluoromethyl radical; and
- R' represents:
  - a hydrogen atom,
  - a straight-chain or branched alkyl radical containing from 1 to 5 carbon atoms and optionally containing a double bond, or
  - an aralkyl radical in which the alkyl moiety contains from 1 to 5 carbon atoms;

in racemic form and in the form of enantiomers, and their physiologically tolerable salts with appropriate acids,

characterised in that :

A) a substituted morpholine of the general formula II :

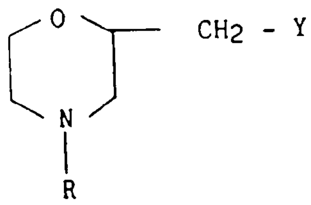

(II)

wherein :

- R is a protecting group, such as a benzyl or trityl group and
- Y is a chlorine or bromine atom or a tosyloxy radical,

is reacted with an alkali metal salt of hydroxyquinoline of the general formula III :

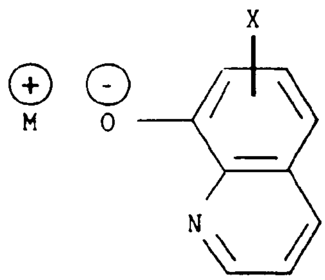

(III)

wherein :

- X is as defined hereinbefore and
- M is an alkali metal, such as sodium or potassium;
- and the compound so obtained of the general formula IV :

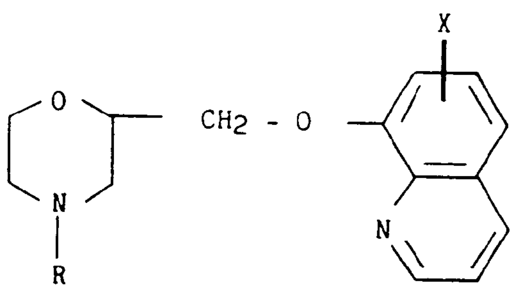

(IV)

wherein R and X are as defined hereinbefore, is reduced

in order to obtain compounds I wherein R' is a hydrogen atom, that is to say compounds corresponding more precisely to the general formula I'

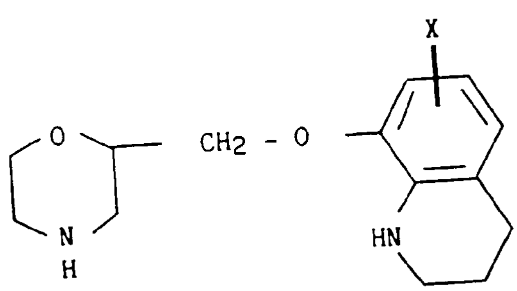

(I')

wherein X is as defined hereinbefore, or

- B) the compound of formula IV defined hereinbefore is reduced to obtain the compound of the general formula V :

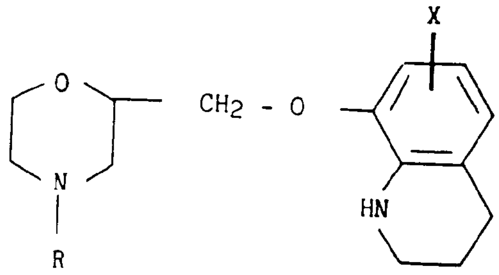

(V)

wherein X and R are as defined hereinbefore,

- the compound V is alkylated
  - . either by means of an aldehyde of formula R"-CHO wherein R" represents: a hydrogen atom
  - . a straight-chain or branched alkyl radical containing from 1 to 4 carbon atoms and optionally containing a double bond, or
  - . an aralkyl radical in which the alkyl moiety contains from 1 to 4 carbon atoms,

  and by means of $NaBH_3CN$, in a polar aprotic solvent with controlled pH;
  - . or by means of an acid of formula R"-COOH wherein R" is as defined above, and NaBH4 in a polar aprotic solvent, and
- the compound so obtained of the general formula VI :

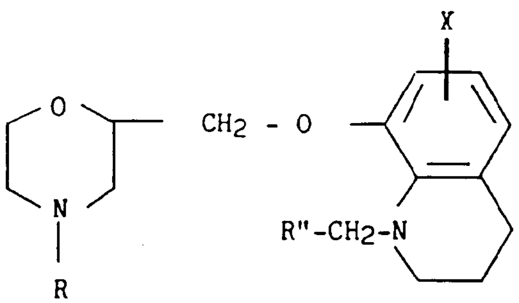

(VI)

wherein X, R and R" are as defined hereinbefore, is debenzylated or detritylated to obtain compounds I wherein R' represents :

- a straight-chain or branched alkyl radical containing from 1 to 5 carbon atoms and optionally containing a double bond, or
- an aralkyl radical in which the alkyl moiety contains from 1 to 5 carbon atoms,

that is to say, compounds corresponding more precisely to the general formula I" :

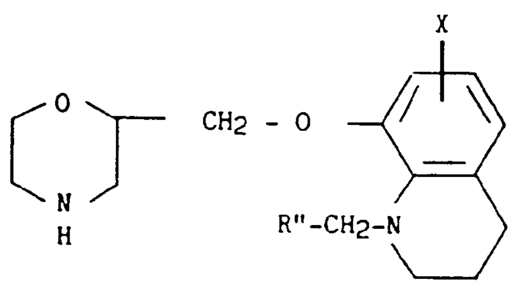

(I")

wherein X and R" are as defined hereinbefore,

- and, if desired, the compounds I' and I" obtained above are treated with appropriate acids to yield the corresponding acid addition salts.

2. A preparation process according to claim 1, characterised in that the condensation of compounds II and III is carried out in an aprotic solvent.

3. A preparation process according to claim 1, characterised in that the condensation of compound II with

compound III in the form of the potassium or sodium salt is carried out by solid-liquid phase transfer in an aprotic solvent, in the presence of a quaternary ammonium salt having a high molecular weight, at a temperature of from 90 to 120°C.

**4.** A preparation process according to any one of claims 1 to 3, characterised in that the reduction of compound IV wherein R is a benzyl radical is carried out by means of hydrogen at reduced pressure, in the presence of a catalyst belonging to the metals of group VIII in a polar solvent, at a temperature of from 20 to 60°C.

**5.** A preparation process according to any one of claims 1 to 3, characterised in that the reduction is effected starting from a compound of formula IV wherein R represents a trityl radical which, by means of hydrolysis carried out using an acid, yields a compound of formula IV wherein R has been replaced by a hydrogen atom, and the latter compound is reduced by a borohydride, in a polar solvent, at a pH of from 6 to 7.